# EUROPEAN PATENT SPECIFICATION

(11) **EP 2 817 323 B1**
(45) Date of publication and mention of the grant of the patent: **06.04.2016**
(21) Application number: 13717558.4
(22) Date of filing: 22.02.2013
(51) Int. Cl.: A01N 37/44, A01N 37/46, A01N 43/713, A01N 43/90, A01N 63/02, C07K 7/64, C07K 7/66

(54) **ANTIMICROBIAL CYCLIC PEPTIDE COMPOSITIONS FOR PLANTS**
ANTIMIKROBISCHE, ZYKLISCHE PEPTIDE ENTHALTENDE ZUSAMMENFASSUNG, FÜR VERWENDUNG IN PFLANZEN GEEIGNET
COMPOSITIONS ANTIMICROBIALES CONTENANT PEPTIDES CYCLIQUES POUR UTILISATION SUR LES PLANTES

(30) Priority: 22.02.2012 ZA 201201316; 22.02.2012 ZA 201201317
(43) Date of publication of application: 31.12.2014
(73) Proprietor: Stellenbosch University, 7600 Western Cape Province (ZA)
(72) Inventor: RAUTENBACH, Marina, Baden Powell Drive Stellenbosch 7600 (ZA); DE BEER, Abré, 7580 Western Cape (ZA); TROSKIE, Anscha Mari, 7600 Western Cape (ZA); VOSLOO, Johan Arnold, Stellenbosch 7600 (ZA)
(74) Representative: Matthews, Derek Peter
(86) International application number: PCT/IB2013/051457
(87) International publication number: WO 2013/150394

(56) References cited:
- WO-A1-2007/074185
- DE-A1- 19 754 298
- M W HAGGAG: "Isolation of bioactive antibiotic peptides from Bacillus brevis and Bacillus polymyxa against Botrytis grey mould in strawberry", ARCHIVES OF PHYTOPATHOLOGY & PLANT PROTECTION, vol. 41, no. 7, 2008, pages 477-491, London

### SEQUENCE LISTING

<110> Stellenbosch University
<120> Antimicrobial Peptide Compositions for Plants
<130> 4.121485
<150> EP13717558.4
   <151> 2013-02-22
<150> ZA 2012/01316
   <151> 2012-02-22
<150> ZA 2012/01317
   <151> 2012-02-22
<160> 187
<170> PatentIn version 3.5
<210> 1
   <211> 10
   <212> PRT
   <213> Artificial Sequence
<220>
   <223> Artificial construct; Cyclic polypeptide
<220>
   <221> VARIANT
   <222> (2)..(2)
   <223> X1 = O or K
<220>
   <221> VARIANT
   <222> (6)..(6)
   <223> X2 = V, L, I, F, W or Y
<220>
   <221> VARIANT
   <222> (7).. (7)
   <223> X3 = v, L, I, F, W, Y, O or K
<220>
   <221> VARIANT
   <222> (8)..(8)
   <223> X4 = N, Q or L
<220>
   <221> VARIANT
   <222> (9)..(9)
   <223> X5 = Q, v, L or I
<220>
   <221> VARIANT
   <222> (10)..(10)
   <223> X6 = Y, F, W or P
<400> 1
   Val Xaa Leu Phe Pro Xaa Xaa Xaa Xaa Xaa
   1               5                   10
<210> 2
   <211> 10
   <212> PRT
   <213> Artificial Sequence
<220>
   <223> Artificial construct; Cyclic polypeptide
<220>
   <221> VARIANT
   <222> (2)..(2)
   <223> X1 = O or K
<220>
   <221> VARIANT
   <222> (6)..(6)
   <223> X7 = W or F
<220>
   <221> VARIANT
   <222> (7)..(7)
   <223> X8 = w or f
<220>
   <221> VARIANT
   <222> (10)..(10)
   <223> X9 = Y, W, or F
<400> 2
   Val Xaa Leu Phe Pro Xaa Xaa Asn Gln Xaa
   1               5                   10
<210> 3
   <211> 10
   <212> PRT
   <213> Artificial Sequence
<220>
   <223> Artificial construct; Cyclic polypeptide
<220>
   <221> VARIANT
   <222> (2)..(2)
   <223> X1 = O or K
<220>
   <221> VARIANT
   <222> (7)..(7)
   <223> X1 = O or K
<400> 3
   Val Xaa Leu Phe Pro Val Xaa Leu Phe Pro
   1               5                   10
<210> 4
   <211> 5
   <212> PRT
   <213> Artificial Sequence
<220>
   <223> Artificial construct; Cyclic polypeptide
<220>
   <221> MOD_RES
   <222> (2)..(2)
   <223> Orn
<400> 4
   Val Xaa Leu Phe Pro
   1 5
<210> 5
   <211> 5
   <212> PRT
   <213> Artificial Sequence
<220>
   <223> Artificial construct; Cyclic polypeptide
<400> 5
   Pro Phe Asn Gln Tyr
   1 5
<210> 6
   <211> 10
   <212> PRT
   <213> Bacillus aneurinolyticus
<400> 6
   Val Lys Leu Phe Pro Trp Trp Asn Gln Tyr
   1 5 10
<210> 7
   <211> 10
   <212> PRT
   <213> Bacillus aneurinolyticus
<220>
   <221> MOD_RES
   <222> (2)..(2)
   <223> Orn
<400> 7
   Val Xaa Leu Phe Pro Trp Trp Asn Gln Tyr
   1 5 10
<210> 8
   <211> 10
   <212> PRT
   <213> Bacillus aneurinolyticus
<400> 8
   Val Lys Leu Phe Pro Trp Phe Asn Gln Tyr
   1 5 10
<210> 9
   <211> 10
   <212> PRT
   <213> Bacillus aneurinolyticus
<220>
   <221> MOD_RES
   <222> (2)..(2)
   <223> Orn
<400> 9
   Val Xaa Leu Phe Pro Trp Phe Asn Gln Tyr
   1 5 10
<210> 10
   <211> 10
   <212> PRT
   <213> Bacillus aneurinolyticus
<400> 10
   Val Lys Leu Phe Pro Phe Trp Asn Gln Tyr
   1 5 10
<210> 11
   <211> 10
   <212> PRT
   <213> Bacillus aneurinolyticus
<220>
   <221> MOD_RES
   <222> (2)..(2)
   <223> Orn
<400> 11
   Val Xaa Leu Phe Pro Phe Trp Asn Gln Tyr
   1 5 10
<210> 12
   <211> 10
   <212> PRT
   <213> Bacillus aneurinolyticus
<400> 12
   Val Lys Leu Phe Pro Phe Phe Asn Gln Tyr
   1 5 10
<210> 13
   <211> 10
   <212> PRT
   <213> Bacillus aneurinolyticus
<220>
   <221> MOD_RES
   <222> (2)..(2)
   <223> Orn
<400> 13
   Val Xaa Leu Phe Pro Phe Phe Asn Gln Tyr
   1 5 10
<210> 14
   <211> 10
   <212> PRT
   <213> Bacillus aneurinolyticus
<400> 14
   Val Lys Leu Phe Pro Tyr Trp Asn Gln Tyr
   1 5 10
<210> 15
   <211> 10
   <212> PRT
   <213> Bacillus aneurinolyticus
<220>
   <221> MOD_RES
   <222> (2)..(2)
   <223> Orn
<400> 15
   Val Xaa Leu Phe Pro Tyr Trp Asn Gln Tyr
   1 5 10
<210> 16
   <211> 10
   <212> PRT
   <213> Bacillus aneurinolyticus
<400> 16
   Val Lys Leu Phe Pro Tyr Phe Asn Gln Tyr
   1 5 10
<210> 17
   <211> 10
   <212> PRT
   <213> Bacillus aneurinolyticus
<220>
   <221> MOD_RES
   <222> (2)..(2)
   <223> Orn
<400> 17
   Val Xaa Leu Phe Pro Tyr Phe Asn Gln Tyr
   1 5 10
<210> 18
   <211> 10
   <212> PRT
   <213> Bacillus aneurinolyticus
<400> 18
   Val Lys Leu Phe Pro Phe Tyr Asn Gln Tyr
   1 5 10
<210> 19
   <211> 10
   <212> PRT
   <213> Bacillus aneurinolyticus
<220>
   <221> MOD_RES
   <222> (2)..(2)
   <223> Orn
<400> 19
   Val Xaa Leu Phe Pro Phe Tyr Asn Gln Tyr
   1 5 10
<210> 20
   <211> 10
   <212> PRT
   <213> Artificial Sequence
<220>
   <223> Artificial construct; Cyclic polypeptide
<400> 20
   Val Lys Leu Phe Pro Trp Tyr Asn Gln Tyr
   1 5 10
<210> 21
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 21
   Val Xaa Leu Phe Pro Trp Tyr Asn Gln Tyr
   1 5 10
<210> 22
   <211> 10
   <212> PRT
   <213> Artificial Sequence
<220>
   <223> Artificial construct; Cyclic polypeptide
<400> 22
   Leu Lys Leu Phe Pro Trp Trp Asn Gln Tyr
   1 5 10
<210> 23
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 23
   Leu Xaa Leu Phe Pro Trp Trp Asn Gln Tyr
   1 5 10
<210> 24
   <211> 10
   <212> PRT
   <213> Bacillus aneurinolyticus
<400> 24
   Leu Lys Leu Phe Pro Trp Phe Asn Gln Tyr
   1 5 10
<210> 25
   <211> 10
   <212> PRT
   <213> Bacillus aneurinolyticus
<220>
   <221> MOD_RES
   <222> (2)..(2)
   <223> Orn
<400> 25
   Leu Xaa Leu Phe Pro Trp Phe Asn Gln Tyr
   1 5 10
<210> 26
   <211> 10
   <212> PRT
   <213> Artificial Sequence
<220>
   <223> Artificial construct; Cyclic polypeptide
<400> 26
   Leu Lys Leu Phe Pro Phe Trp Asn Gln Tyr
   1 5 10
<210> 27
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 27
   Leu Xaa Leu Phe Pro Phe Trp Asn Gln Tyr
   1 5 10
<210> 28
   <211> 10
   <212> PRT
   <213> Artificial Sequence
<220>
   <223> Artificial construct; Cyclic polypeptide
<400> 28
   Leu Lys Leu Phe Pro Phe Phe Asn Gln Tyr
   1 5 10
<210> 29
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 29
   Leu Xaa Leu Phe Pro Phe Phe Asn Gln Tyr
   1 5 10
<210> 30
   <211> 10
   <212> PRT
   <213> Artificial Sequence
<220>
   <223> Artificial construct; Cyclic polypeptide
<400> 30
   Leu Lys Leu Phe Pro Tyr Trp Asn Gln Tyr
   1 5 10
<210> 31
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 31
   Leu Xaa Leu Phe Pro Tyr Trp Asn Gln Tyr
   1 5 10
<210> 32
   <211> 10
   <212> PRT
   <213> Artificial Sequence
<220>
   <223> Artificial construct; Cyclic polypeptide
<400> 32
   Leu Lys Leu Phe Pro Tyr Phe Asn Gln Tyr
   1 5 10
<210> 33
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 33
   Leu Xaa Leu Phe Pro Tyr Phe Asn Gln Tyr
   1               5                   10
<210> 34
   <211> 10
   <212> PRT
   <213> Artificial Sequence
<220>
   <223> Artificial construct; Cyclic polypeptide
<400> 34
   Leu Lys Leu Phe Pro Phe Tyr Asn Gln Tyr
   1               5                   10
<210> 35
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 35
   Leu Xaa Leu Phe Pro Phe Tyr Asn Gln Tyr
   1               5                   10
<210> 36
   <211> 10
   <212> PRT
   <213> Artificial Sequence
<220>
   <223> Artificial construct; Cyclic polypeptide
<400> 36
   Leu Lys Leu Phe Pro Trp Tyr Asn Gln Tyr
   1 5 10
<210> 37
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 37
   Leu Xaa Leu Phe Pro Trp Tyr Asn Gln Tyr
   1 5 10
<210> 38
   <211> 10
   <212> PRT
   <213> Artificial Sequence
<220>
   <223> Artificial construct; Cyclic polypeptide
<400> 38
   Ile Lys Leu Phe Pro Trp Trp Asn Gln Tyr
   1 5 10
<210> 39
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 39
   Ile Xaa Leu Phe Pro Trp Trp Asn Gln Tyr
   1 5 10
<210> 40
   <211> 10
   <212> PRT
   <213> Bacillus aneurinolyticus
<400> 40
   Ile Lys Leu Phe Pro Trp Phe Asn Gln Tyr
   1 5 10
<210> 41
   <211> 10
   <212> PRT
   <213> Bacillus aneurinolyticus
<220>
   <221> MOD_RES
   <222> (2)..(2)
   <223> Orn
<400> 41
   Ile Xaa Leu Phe Pro Trp Phe Asn Gln Tyr
   1 5 10
<210> 42
   <211> 10
   <212> PRT
   <213> Artificial Sequence
<220>
   <223> Artificial construct; Cyclic polypeptide
<400> 42
   Ile Lys Leu Phe Pro Phe Trp Asn Gln Tyr
   1 5 10
<210> 43
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 43
   Ile Xaa Leu Phe Pro Phe Trp Asn Gln Tyr
   1 5 10
<210> 44
   <211> 10
   <212> PRT
   <213> Artificial Sequence
<220>
   <223> Artificial construct; Cyclic polypeptide
<400> 44
   Ile Lys Leu Phe Pro Phe Phe Asn Gln Tyr
   1 5 10
<210> 45
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 45
   Ile Xaa Leu Phe Pro Phe Phe Asn Gln Tyr
   1 5 10
<210> 46
   <211> 10
   <212> PRT
   <213> Artificial Sequence
<220>
   <223> Artificial construct; Cyclic polypeptide
<400> 46
   Ile Lys Leu Phe Pro Tyr Trp Asn Gln Tyr
   1 5 10
<210> 47
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 47
   Ile Xaa Leu Phe Pro Tyr Trp Asn Gln Tyr
   1 5 10
<210> 48
   <211> 10
   <212> PRT
   <213> Artificial Sequence
<220>
   <223> Artificial construct; Cyclic polypeptide
<400> 48
   Ile Lys Leu Phe Pro Tyr Phe Asn Gln Tyr
   1               5                   10
<210> 49
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 49
   Ile Xaa Leu Phe Pro Tyr Phe Asn Gln Tyr
   1               5                   10
<210> 50
   <211> 10
   <212> PRT
   <213> Artificial Sequence
<220>
   <223> Artificial construct; Cyclic polypeptide
<400> 50
   Ile Lys Leu Phe Pro Phe Tyr Asn Gln Tyr
   1               5                   10
<210> 51
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 51
   Ile Xaa Leu Phe Pro Phe Tyr Asn Gln Tyr
   1 5 10
<210> 52
   <211> 10
   <212> PRT
   <213> Artificial Sequence
<220>
   <223> Artificial construct; Cyclic polypeptide
<400> 52
   Ile Lys Leu Phe Pro Trp Tyr Asn Gln Tyr
   1 5 10
<210> 53
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 53
   Ile Xaa Leu Phe Pro Trp Tyr Asn Gln Tyr
   1 5 10
<210> 54
   <211> 10
   <212> PRT
   <213> Artificial Sequence
<220>
   <223> Artificial construct; Cyclic polypeptide
<400> 54
   Val Lys Leu Phe Pro Leu Trp Asn Gln Tyr
   1 5 10
<210> 55
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 55
   Val Xaa Leu Phe Pro Leu Trp Asn Gln Tyr
   1 5 10
<210> 56
   <211> 10
   <212> PRT
   <213> Bacillus aneurinolyticus
<400> 56
   Val Lys Leu Phe Pro Leu Phe Asn Gln Tyr
   1 5 10
<210> 57
   <211> 10
   <212> PRT
   <213> Bacillus aneurinolyticus
<220>
   <221> MOD_RES
   <222> (2)..(2)
   <223> Orn
<400> 57
   Val Xaa Leu Phe Pro Leu Phe Asn Gln Tyr
   1 5 10
<210> 58
   <211> 10
   <212> PRT
   <213> Artificial Sequence
<220>
   <223> Artificial construct; Cyclic polypeptide
<400> 58
   Val Lys Leu Phe Pro Leu Tyr Asn Gln Tyr
   1 5 10
<210> 59
   <211> 10
   <212> PRT
   <213> Artificial Sequence
<220>
   <223> Artificial construct; Cyclic polypeptide
<220>
<221> MOD_RES
   <222> (2)..(2)
   <223> Orn
<400> 59
   Val Xaa Leu Phe Pro Leu Tyr Asn Gln Tyr
   1 5 10
<210> 60
   <211> 10
   <212> PRT
   <213> Bacillus aneurinolyticus
<400> 60
   Val Lys Leu Phe Pro Trp Trp Asn Gln Trp
   1 5 10
<210> 61
   <211> 10
   <212> PRT
   <213> Bacillus aneurinolyticus
<220>
   <221> MOD_RES
   <222> (2)..(2)
   <223> Orn
<400> 61
   Val Xaa Leu Phe Pro Trp Trp Asn Gln Trp
   1 5 10
<210> 62
   <211> 10
   <212> PRT
   <213> Bacillus aneurinolyticus
<400> 62
   Val Lys Leu Phe Pro Trp Phe Asn Gln Trp
   1 5 10
<210> 63
   <211> 10
   <212> PRT
   <213> Bacillus aneurinolyticus
<220>
   <221> MOD_RES
   <222> (2)..(2)
   <223> Orn
<400> 63
   Val Xaa Leu Phe Pro Trp Phe Asn Gln Trp
   1 5 10
<210> 64
   <211> 10
   <212> PRT
   <213> Artificial Sequence
<220>
   <223> Artificial construct; Cyclic polypeptide
<400> 64
   Val Lys Leu Phe Pro Phe Trp Asn Gln Trp
   1 5 10
<210> 65
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 65
   Val Xaa Leu Phe Pro Phe Trp Asn Gln Trp
   1 5 10
<210> 66
   <211> 10
   <212> PRT
   <213> Artificial Sequence
<220>
   <223> Artificial construct; Cyclic polypeptide
<400> 66
   Val Lys Leu Phe Pro Phe Phe Asn Gln Trp
   1 5 10
<210> 67
   <211> 10
   <212> PRT
   <213> Bacillus aneurinolyticus
<220>
   <221> MOD_RES
   <222> (2)..(2)
   <223> Orn
<400> 67
   Val Xaa Leu Phe Pro Phe Phe Asn Gln Trp
   1 5 10
<210> 68
   <211> 10
   <212> PRT
   <213> Artificial Sequence
<220>
   <223> Artificial construct; Cyclic polypeptide
<400> 68
   Val Lys Leu Phe Pro Tyr Trp Asn Gln Trp
   1 5 10
<210> 69
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 69
   Val Xaa Leu Phe Pro Tyr Trp Asn Gln Trp
   1 5 10
<210> 70
   <211> 10
   <212> PRT
   <213> Artificial Sequence
<220>
   <223> Artificial construct; Cyclic polypeptide
<400> 70
   Val Lys Leu Phe Pro Tyr Phe Asn Gln Trp
   1 5 10
<210> 71
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 71
   Val Xaa Leu Phe Pro Tyr Phe Asn Gln Trp
   1 5 10
<210> 72
   <211> 10
   <212> PRT
   <213> Artificial Sequence
<220>
   <223> Artificial construct; Cyclic polypeptide
<400> 72
   Val Lys Leu Phe Pro Phe Tyr Asn Gln Trp
   1 5 10
<210> 73
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 73
   Val Xaa Leu Phe Pro Phe Tyr Asn Gln Trp
   1 5 10
<210> 74
   <211> 10
   <212> PRT
   <213> Artificial Sequence
<220>
   <223> Artificial construct; Cyclic polypeptide
<400> 74
   Val Lys Leu Phe Pro Trp Tyr Asn Gln Trp
   1 5 10
<210> 75
   <211> 10
   <212> PRT
   <213> Artificial Sequence
   <220>
   <223> Artificial construct; Cyclic polypeptide
<220>
   <221> MOD_RES
   <222> (2)..(2)
   <223> Orn
<400> 75
   Val Xaa Leu Phe Pro Trp Tyr Asn Gln Trp
   1 5 10
<210> 76
   <211> 10
   <212> PRT
   <213> Artificial Sequence
<220>
   <223> Artificial construct; Cyclic polypeptide
<400> 76
   Leu Lys Leu Phe Pro Trp Trp Asn Gln Trp
   1 5 10
<210> 77
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 77
   Leu Xaa Leu Phe Pro Trp Trp Asn Gln Trp
   1 5 10
<210> 78
   <211> 10
   <212> PRT
   <213> Artificial Sequence
<220>
   <223> Artificial construct; Cyclic polypeptide
<400> 78
   Leu Lys Leu Phe Pro Trp Phe Asn Gln Trp
   1 5 10
<210> 79
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 79
   Leu Xaa Leu Phe Pro Trp Phe Asn Gln Trp
   1 5 10
<210> 80
   <211> 10
   <212> PRT
   <213> Artificial Sequence
<220>
   <223> Artificial construct; Cyclic polypeptide
<400> 80
   Leu Lys Leu Phe Pro Phe Trp Asn Gln Trp
   1 5 10
<210> 81
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 81
   Leu Xaa Leu Phe Pro Phe Trp Asn Gln Trp
   1 5 10
<210> 82
   <211> 10
   <212> PRT
   <213> Artificial Sequence
<220>
   <223> Artificial construct; Cyclic polypeptide
<400> 82
   Leu Lys Leu Phe Pro Phe Phe Asn Gln Trp
   1 5 10
<210> 83
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 83
   Leu Xaa Leu Phe Pro Phe Phe Asn Gln Trp
   1 5 10
<210> 84
   <211> 10
   <212> PRT
   <213> Artificial Sequence
<220>
   <223> Artificial construct; Cyclic polypeptide
<400> 84
   Leu Lys Leu Phe Pro Tyr Trp Asn Gln Trp
   1 5 10
<210> 85
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 85
   Leu Xaa Leu Phe Pro Tyr Trp Asn Gln Trp
   1 5 10
<210> 86
   <211> 10
   <212> PRT
   <213> Artificial Sequence
<220>
   <223> Artificial construct; Cyclic polypeptide
<400> 86
   Leu Lys Leu Phe Pro Tyr Phe Asn Gln Trp
   1 5 10
<210> 87
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 87
   Leu Xaa Leu Phe Pro Tyr Phe Asn Gln Trp
   1 5 10
<210> 88
   <211> 10
   <212> PRT
   <213> Artificial Sequence
<220>
   <223> Artificial construct; Cyclic polypeptide
<400> 88
   Leu Lys Leu Phe Pro Phe Tyr Asn Gln Trp
   1 5 10
<210> 89
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 89
   Leu Xaa Leu Phe Pro Phe Tyr Asn Gln Trp
   1 5 10
<210> 90
   <211> 10
   <212> PRT
   <213> Artificial Sequence
<220>
   <223> Artificial construct; Cyclic polypeptide
<400> 90
   Leu Lys Leu Phe Pro Trp Tyr Asn Gln Trp
   1 5 10
<210> 91
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 91
   Leu Xaa Leu Phe Pro Trp Tyr Asn Gln Trp
   1 5 10
<210> 92
   <211> 10
   <212> PRT
   <213> Artificial Sequence
<220>
   <223> Artificial construct; Cyclic polypeptide
<400> 92
   Ile Lys Leu Phe Pro Trp Trp Asn Gln Trp
   1 5 10
<210> 93
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 93
   Ile Xaa Leu Phe Pro Trp Trp Asn Gln Trp
   1               5                   10
<210> 94
   <211> 10
   <212> PRT
   <213> Artificial Sequence
<220>
   <223> Artificial construct; Cyclic polypeptide
<400> 94
   Ile Lys Leu Phe Pro Trp Phe Asn Gln Trp
   1               5                   10
<210> 95
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 95
   Ile Xaa Leu Phe Pro Trp Phe Asn Gln Trp
   1               5                   10
<210> 96
   <211> 10
   <212> PRT
   <213> Artificial Sequence
<220>
   <223> Artificial construct; Cyclic polypeptide
<400> 96
   Ile Lys Leu Phe Pro Phe Trp Asn Gln Trp
   1 5 10
<210> 97
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 97
   Ile Xaa Leu Phe Pro Phe Trp Asn Gln Trp
   1 5 10
<210> 98
   <211> 10
   <212> PRT
   <213> Artificial Sequence
<220>
   <223> Artificial construct; Cyclic polypeptide
<400> 98
   Ile Lys Leu Phe Pro Phe Phe Asn Gln Trp
   1 5 10
<210> 99
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 99
   Ile Xaa Leu Phe Pro Phe Phe Asn Gln Trp
   1 5 10
<210> 100
   <211> 10
   <212> PRT
   <213> Artificial Sequence
<220>
   <223> Artificial construct; Cyclic polypeptide
<400> 100
   Ile Lys Leu Phe Pro Tyr Trp Asn Gln Trp
   1 5 10
<210> 101
   <211> 10
   <212> PRT
   <213> Artificial Sequence
   <220>
   <223> Artificial construct; Cyclic polypeptide
<220>
   <221> MOD_RES
   <222> (2)..(2)
   <223> Orn
<400> 101
   Ile Xaa Leu Phe Pro Tyr Trp Asn Gln Trp
   1 5 10
<210> 102
   <211> 10
   <212> PRT
   <213> Artificial Sequence
<220>
   <223> Artificial construct; Cyclic polypeptide
<400> 102
   Ile Lys Leu Phe Pro Tyr Phe Asn Gln Trp
   1 5 10
<210> 103
   <211> 10
   <212> PRT
   <213> Artificial Sequence
'<220>
   <223> Artificial construct; Cyclic polypeptide
<220>
   <221> MOD_RES
   <222> (2)..(2)
   <223> Orn
<400> 103
   Ile Xaa Leu Phe Pro Tyr Phe Asn Gln Trp
   1 5 10
<210> 104
   <211> 10
   <212> PRT
   <213> Artificial Sequence
<220>
   <223> Artificial construct; Cyclic polypeptide
<400> 104
   Ile Lys Leu Phe Pro Phe Tyr Asn Gln Trp
   1 5 10
<210> 105
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 105
   Ile Xaa Leu Phe Pro Phe Tyr Asn Gln Trp
   1 5 10
<210> 106
   <211> 10
   <212> PRT
   <213> Artificial Sequence
<220>
   <223> Artificial construct; Cyclic polypeptide
<400> 106
   Ile Lys Leu Phe Pro Trp Tyr Asn Gln Trp
   1 5 10
<210> 107
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 107
   Ile Xaa Leu Phe Pro Trp Tyr Asn Gln Trp
   1 5 10
<210> 108
   <211> 10
   <212> PRT
   <213> Artificial Sequence
<220>
   <223> Artificial construct; Cyclic polypeptide
<400> 108
   Val Lys Leu Phe Pro Leu Trp Asn Gln Trp
   1 5 10
<210> 109
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 109
   Val Xaa Leu Phe Pro Leu Trp Asn Gln Trp
   1 5 10
<210> 110
   <211> 10
   <212> PRT
   <213> Artificial Sequence
<220>
   <223> Artificial construct; Cyclic polypeptide
<400> 110
   Val Lys Leu Phe Pro Leu Phe Asn Gln Trp
   1 5 10
<210> 111
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 111
   Val Xaa Leu Phe Pro Leu Phe Asn Gln Trp
   1               5                   10
<210> 112
   <211> 10
   <212> PRT
   <213> Artificial Sequence
<220>
   <223> Artificial construct; Cyclic polypeptide
<400> 112
   Val Lys Leu Phe Pro Leu Tyr Asn Gln Trp
   1               5                   10
<210> 113
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 113
   Val Xaa Leu Phe Pro Leu Tyr Asn Gln Trp
   1               5                   10
<210> 114
   <211> 10
   <212> PRT
   <213> Artificial Sequence
<220>
   <223> Artificial construct; Cyclic polypeptide
<400> 114
   Val Lys Leu Phe Pro Trp Trp Asn Gln Phe
   1 5 10
<210> 115
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 115
   Val Xaa Leu Phe Pro Trp Trp Asn Gln Phe
   1 5 10
<210> 116
   <211> 10
   <212> PRT
   <213> Artificial Sequence
<220>
   <223> Artificial construct; Cyclic polypeptide
<400> 116
   Val Lys Leu Phe Pro Trp Phe Asn Gln Phe
   1 5 10
<210> 117
   <211> 10
   <212> PRT
   <213> Bacillus aneurinolyticus
<220>
   <221> MOD_RES
   <222> (2)..(2)
   <223> Orn
<400> 117
   Val Xaa Leu Phe Pro Trp Phe Asn Gln Phe
   1 5 10
<210> 118
   <211> 10
   <212> PRT
   <213> Artificial Sequence
<220>
   <223> Artificial construct; Cyclic polypeptide
<400> 118
   Val Lys Leu Phe Pro Phe Trp Asn Gln Phe
   1 5 10
<210> 119
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 119
   Val Xaa Leu Phe Pro Phe Trp Asn Gln Phe
   1 5 10
<210> 120
   <211> 10
   <212> PRT
   <213> Artificial Sequence
<220>
   <223> Artificial construct; Cyclic polypeptide
<400> 120
   Val Lys Leu Phe Pro Phe Phe Asn Gln Phe
   1 5 10
<210> 121
   <211> 10
   <212> PRT
   <213> Bacillus aneurinolyticus
<220>
   <221> MOD_RES
   <222> (2)..(2)
   <223> Orn
<400> 121
   Val Xaa Leu Phe Pro Phe Phe Asn Gln Phe
   1 5 10
<210> 122
   <211> 10
   <212> PRT
   <213> Artificial Sequence
<220>
   <223> Artificial construct; Cyclic polypeptide
<400> 122
   Val Lys Leu Phe Pro Tyr Trp Asn Gln Phe
   1               5                   10
<210> 123
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 123
   Val Xaa Leu Phe Pro Tyr Trp Asn Gln Phe
   1               5                   10
<210> 124
   <211> 10
   <212> PRT
   <213> Artificial Sequence
<220>
   <223> Artificial construct; Cyclic polypeptide
<400> 124
   Val Lys Leu Phe Pro Tyr Phe Asn Gln Phe
   1               5                   10
<210> 125
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 125
   Val Xaa Leu Phe Pro Tyr Phe Asn Gln Phe
   1 5 10
<210> 126
   <211> 10
   <212> PRT
   <213> Artificial Sequence
<220>
   <223> Artificial construct; Cyclic polypeptide
<400> 126
   Val Lys Leu Phe Pro Phe Tyr Asn Gln Phe
   1 5 10
<210> 127
   <211> 10
   <212> PRT
   <213> Artificial Sequence
<220>
   <223> Artificial construct; Cyclic polypeptide
   <220>
   <221> MOD_RES
   <222> (2)..(2)
   <223> Orn
<400> 127
   Val Xaa Leu Phe Pro Phe Tyr Asn Gln Phe
   1 5 10
<210> 128
   <211> 10
   <212> PRT
   <213> Artificial Sequence
<220>
   <223> Artificial construct; Cyclic polypeptide
<400> 128
   Val Lys Leu Phe Pro Trp Tyr Asn Gln Phe
   1 5 10
<210> 129
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 129
   Val Xaa Leu Phe Pro Trp Tyr Asn Gln Phe
   1 5 10
<210> 130
   <211> 10
   <212> PRT
   <213> Artificial Sequence
<220>
   <223> Artificial construct; Cyclic polypeptide
<400> 130
   Leu Lys Leu Phe Pro Trp Trp Asn Gln Phe
   1 5 10
<210> 131
   <211> 10
   <212> PRT
   <213> Artificial Sequence
   <220>
   <223> Artificial construct; Cyclic polypeptide
<220>
   <221> MOD_RES
   <222> (2)..(2)
   <223> Orn
<400> 131
   Leu Xaa Leu Phe Pro Trp Trp Asn Gln Phe
   1 5 10
<210> 132
   <211> 10
   <212> PRT
   <213> Artificial Sequence
<220>
   <223> Artificial construct; Cyclic polypeptide
<400> 132
   Leu Lys Leu Phe Pro Trp Phe Asn Gln Phe
   1 5 10
<210> 133
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 133
   Leu Xaa Leu Phe Pro Trp Phe Asn Gln Phe
   1 5 10
<210> 134
   <211> 10
   <212> PRT
   <213> Artificial Sequence
<220>
   <223> Artificial construct; Cyclic polypeptide
<400> 134
   Leu Lys Leu Phe Pro Phe Trp Asn Gln Phe
   1 5 10
<210> 135
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 135
   Leu Xaa Leu Phe Pro Phe Trp Asn Gln Phe
   1 5 10
<210> 136
   <211> 10
   <212> PRT
   <213> Artificial Sequence
<220>
   <223> Artificial construct; Cyclic polypeptide
<400> 136
   Leu Lys Leu Phe Pro Tyr Trp Asn Gln Phe
   1 5 10
<210> 137
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 137
   Leu Xaa Leu Phe Pro Tyr Trp Asn Gln Phe
   1               5                   10
<210> 138
   <211> 10
   <212> PRT
   <213> Artificial Sequence
<220>
   <223> Artificial construct; Cyclic polypeptide
<400> 138
   Leu Lys Leu Phe Pro Tyr Phe Asn Gln Phe
   1               5                   10
<210> 139
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 139
   Leu Xaa Leu Phe Pro Tyr Phe Asn Gln Phe
   1               5                   10
<210> 140
   <211> 10
   <212> PRT
   <213> Artificial Sequence
<220>
   <223> Artificial construct; Cyclic polypeptide
<400> 140
   Leu Lys Leu Phe Pro Phe Tyr Asn Gln Phe
   1 5 10
<210> 141
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 141
   Leu Xaa Leu Phe Pro Phe Tyr Asn Gln Phe
   1 5 10
<210> 142
   <211> 10
   <212> PRT
   <213> Artificial Sequence
<220>
   <223> Artificial construct; Cyclic polypeptide
<400> 142
   Leu Lys Leu Phe Pro Trp Tyr Asn Gln Phe
   1 5 10
<210> 143
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 143
   Leu Xaa Leu Phe Pro Trp Tyr Asn Gln Phe
   1 5 10
<210> 144
   <211> 10
   <212> PRT
   <213> Artificial Sequence
<220>
   <223> Artificial construct; Cyclic polypeptide
<400> 144
   Leu Lys Leu Phe Pro Phe Phe Asn Gln Phe
   1               5                   10
<210> 145
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 145
   Leu Xaa Leu Phe Pro Phe Phe Asn Gln Phe
   1               5                   10
<210> 146
   <211> 10
   <212> PRT
   <213> Artificial Sequence
<220>
   <223> Artificial construct; Cyclic polypeptide
<400> 146
   Ile Lys Leu Phe Pro Trp Trp Asn Gln Phe
   1               5                   10
<210> 147
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 147
   Ile Xaa Leu Phe Pro Trp Trp Asn Gln Phe
   1 5 10
<210> 148
   <211> 10
   <212> PRT
   <213> Artificial Sequence
<220>
   <223> Artificial construct; Cyclic polypeptide
<400> 148
   Ile Lys Leu Phe Pro Trp Phe Asn Gln Phe
   1 5 10
<210> 149
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 149
   Ile Xaa Leu Phe Pro Trp Phe Asn Gln Phe
   1 5 10
<210> 150
   <211> 10
   <212> PRT
   <213> Artificial Sequence
<220>
   <223> Artificial construct; Cyclic polypeptide
<400> 150
   Ile Lys Leu Phe Pro Phe Trp Asn Gln Phe
   1 5 10
<210> 151
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 151
   Ile Xaa Leu Phe Pro Phe Trp Asn Gln Phe
   1               5                   10
<210> 152
   <211> 10
   <212> PRT
   <213> Artificial Sequence
<220>
   <223> Artificial construct; Cyclic polypeptide
<400> 152
   Ile Lys Leu Phe Pro Tyr Trp Asn Gln Phe
   1               5                   10
<210> 153
   <211> 10
   <212> PRT
   <213> Artificial Sequence
<220>
   <223> Artificial construct; Cyclic polypeptide
   <220>
   <221> MOD_RES
   <222> (2)..(2)
   <223> Orn
<400> 153
   Ile Xaa Leu Phe Pro Tyr Trp Asn Gln Phe
   1               5                   10
<210> 154
   <211> 10
   <212> PRT
   <213> Artificial Sequence
<220>
   <223> Artificial construct; Cyclic polypeptide
<400> 154
   Ile Lys Leu Phe Pro Tyr Phe Asn Gln Phe
   1               5                   10
<210> 155
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 155
   Ile Xaa Leu Phe Pro Tyr Phe Asn Gln Phe
   1 5 10
<210> 156
   <211> 10
   <212> PRT
   <213> Artificial Sequence
<220>
   <223> Artificial construct; Cyclic polypeptide
<400> 156
   Ile Lys Leu Phe Pro Phe Tyr Asn Gln Phe
   1 5 10
<210> 157
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 157
   Ile Xaa Leu Phe Pro Phe Tyr Asn Gln Phe
   1 5 10
<210> 158
   <211> 10
   <212> PRT
   <213> Artificial Sequence.
<220>
   <223> Artificial construct; Cyclic polypeptide
<400> 158
   Ile Lys Leu Phe Pro Trp Tyr Asn Gln Phe
   1 5 10
<210> 159
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 159
   Ile Xaa Leu Phe Pro Trp Tyr Asn Gln Phe
   1               5                   10
<210> 160
   <211> 10
   <212> PRT
   <213> Artificial Sequence
<220>
   <223> Artificial construct; Cyclic polypeptide
<400> 160
   Ile Lys Leu Phe Pro Phe Phe Asn Gln Phe
   1               5                   10
<210> 161
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 161
   Ile Xaa Leu Phe Pro Phe Phe Asn Gln Phe
   1               5                   10
<210> 162
   <211> 10
   <212> PRT
   <213> Artificial Sequence
<220>
   <223> Artificial construct; Cyclic polypeptide
<400> 162
   Val Lys Leu Phe Pro Leu Trp Asn Gln Phe
   1 5 10
<210> 163
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 163
   Val Xaa Leu Phe Pro Leu Trp Asn Gln Phe
   1 5 10
<210> 164
   <211> 10
   <212> PRT
   <213> Artificial Sequence
<220>
   <223> Artificial construct; Cyclic polypeptide
<400> 164
   Val Lys Leu Phe Pro Leu Phe Asn Gln Phe
   1 5 10
<210> 165
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 165
   Val Xaa Leu Phe Pro Leu Phe Asn Gln Phe
   1 5 10
<210> 166
   <211> 10
   <212> PRT
   <213> Artificial Sequence
<220>
   <223> Artificial construct; Cyclic polypeptide
<400> 166
   Val Lys Leu Phe Pro Leu Tyr Asn Gln Phe
   1               5                   10
<210> 167
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 167
   Val Xaa Leu Phe Pro Leu Tyr Asn Gln Phe
   1               5                   10
<210> 168
   <211> 10
   <212> PRT
   <213> Aneurinibacillus migulanus
<220>
   <221> MOD_RES
   <222> (2)..(2)
   <223> Orn
<220>
   <221> MOD_RES
   <222> (7)..(7)
   <223> Orn
<400> 168
   Val Xaa Leu Phe Pro Val Xaa Leu Phe Pro
   1               5                   10
<210> 169
   <211> 10
   <212> PRT
   <213> Aneurinibacillus migulanus
<220>
   <221> MOD_RES
   <222> (7)..(7)
   <223> Orn
<400> 169
   Val Lys Leu Phe Pro Val Xaa Leu Phe Pro
   1 5 10
<210> 170
   <211> 10
   <212> PRT
   <213> Aneurinibacillus migulanus
<400> 170
   Val Lys Leu Phe Pro Val Lys Leu Phe Pro
   1 5 10
<210> 171
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<220>
   <221> MOD_RES
   <222> (7)..(7)
   <223> Orn
<400> 171
   Leu Xaa Leu Phe Pro Val Xaa Leu Phe Pro
   1 5 10
<210> 172
   <211> 10
   <212> PRT
   <213> Artificial Sequence
<220>
   <223> Artificial construct; Cyclic polypeptide
<220>
   <221> MOD_RES
   <222> (7)..(7)
   <223> Orn
<400> 172
   Leu Lys Leu Phe Pro Val Xaa Leu Phe Pro
   1 5 10
<210> 173
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 173
   Leu Xaa Leu Phe Pro Val Lys Leu Phe Pro
   1               5                   10
<210> 174
   <211> 10
   <212> PRT
   <213> Artificial Sequence
<220>
   <223> Artificial construct; Cyclic polypeptide
<400> 174
   Leu Lys Leu Phe Pro Val Lys Leu Phe Pro
   1               5                   10
<210> 175
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<220>
   <221> MOD_RES
   <222> (7)..(7)
   <223> Orn
<400> 175
   Leu Xaa Leu Phe Pro Leu Xaa Leu Phe Pro
   1               5                   10
<210> 176
   <211> 10
   <212> PRT
   <213> Artificial Sequence
<220>
   <223> Artificial construct; Cyclic polypeptide
<220>
   <221> MOD_RES
   <222> (7)..(7)
   <223> Orn
<400> 176
   Leu Lys Leu Phe Pro Leu Xaa Leu Phe Pro
   1 5 10
<210> 177
   <211> 10
   <212> PRT
   <213> Artificial Sequence
<220>
   <223> Artificial construct; Cyclic polypeptide
<400> 177
   Leu Lys Leu Phe Pro Leu Lys Leu Phe Pro
   1 5 10
<210> 178
   <211> 10
   <212> PRT
   <213> Artificial Sequence
<220>
   <223> Artificial construct; Cyclic polypeptide
<220>
   <221> VARIANT
   <222> (2)..(2)
   <223> X = cationic amino acid residue
<220>
   <221> VARIANT
   <222> (6)..(6)
   <223> X = aromatic amino acid residue
<220>
   <221> VARIANT
   <222> (7)..(7)
   <223> X = aromatic D-amino acid residue
<220>
   <221> VARIANT
   <222> (10)..(10)
   <223> X = aromatic amino acid residue
<400> 178
   Val Xaa Leu Phe Pro Xaa Xaa Asn Gln Xaa
   1 5 10
<210> 179
   <211> 10
   <212> PRT
   <213> Artificial Sequence
<220>
   <223> Artificial construct; Cyclic polypeptide
<400> 179
   Val Lys Leu Phe Pro Trp Trp Asn Gln Tyr
   1 5 10
<210> 180
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 180
   Val Xaa Leu Phe Pro Trp Trp Asn Gln Tyr
   1 5 10
<210> 181
   <211> 10
   <212> PRT
   <213> Artificial Sequence
<220>
   <223> Artificial construct; Cyclic polypeptide
<400> 181
   Val Lys Leu Phe Pro Trp Phe Asn Gln Tyr
   1 5 10
<210> 182
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 182
   Val Xaa Leu Phe Pro Trp Phe Asn Gln Tyr
   1 5 10
<210> 183
   <211> 10
   <212> PRT
   <213> Artificial Sequence
<220>
   <223> Artificial construct; Cyclic polypeptide
<400> 183
   Val Lys Leu Phe Pro Phe Phe Asn Gln Tyr
   1 5 10
<210> 184
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 184
   Val Xaa Leu Phe Pro Phe Phe Asn Gln Tyr
   1 5 10
<210> 185
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 185
   Val Xaa Leu Phe Pro Trp Trp Asn Gln Trp
   1 5 10
<210> 186
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<400> 186
   Val Xaa Leu Phe Pro Phe Phe Asn Gln Phe
   1               5                   10
<210> 187
   <211> 10
   <212> PRT
   <213> Artificial Sequence
<220>
   <223> Artificial construct; Cyclic polypeptide
<220>
   <221> MOD_RES
   <222> (2)..(2)
   <223> Orn
<220>
   <221> MOD_RES
   <222> (7)..(7)
   <223> Orn
<400> 187
   Val Xaa Leu Phe Pro Val Xaa Leu Phe Pro
   1               5                   10

## Description

### FIELD OF THE INVENTION

This invention relates to antimicrobial compositions for preventing or controlling microbial growth on plants, plant parts or plant material.

### BACKGROUND OF THE INVENTION

The greatest threat to the fresh produce market is the reduction in yield and quality due to microbial infections, especially fungal pathogens. Pre-harvest infection by microbial pathogens results in a loss of approximately 16% in global food production each year, with a further loss of up to 50% as a consequence of post-harvest infections, particularly in developing countries. This has the potential of threatening global food security (Chakraborty and Newton, 2011, Plant Path. 60, 2-14, Montesinos and Bardaji, 2008, Chem. Biodivers. 5, 1225-1237).

Post-harvest infections, such as those caused by *Penicillium* ssp (blue mould), *Monilinia* spp (brown rot) and particularly *Botrytis cinerea* (grey mould) in grape, strawberries, cherries and tree fruits like pears and apples, are the leading cause of major losses in marketable fruits (Lennox et al. 2003. Plant Dis. 87, 639-644; Wilson et al., 1991 Crop Protection 10, 172-177; Romanazzi, 2010, Fresh produce 4, 111-115). Measures to limit losses during storage include low temperature storage, bio-control and treatments with natural compounds as well as chemical treatments (Zheng et al. 2008. Food Control, 19, 470-474; Romanazzi, 2010, Fresh Produce 4, 111-115).

Another major concern in the agricultural industry is pre-harvest losses that are mostly caused by decline of plant health due to infection. Soil-borne *Fusarium* spp. are associated with vine wilt and root rot and affect many plant species (Di Peitro et al, 2003, Mol. Plant Path 4, 315-325; Berrocal-Lobo and Molina, 2008, Trends Plant Sci. 13, 145-150, Highet and Nair, 1995, Aus. J. Grape Wine Res, 1 48-50). Fungal spores can persist in soil for years and cuttings for planting or leaf detachment can encourage infection through vascular wounds, although infection in many plants takes place through roots (Berrocal-Lobo and Molina, 2008 Trends Plant Sci. 13, 145-150, Highet and Nair, 1995, Aus. J. Grape Wine Res, 1 48-50). However, the two most destructive diseases associated with grapevine decline are black foot disease, caused by *Cylindrocarpon* spp., and young grapevine decline or Petri disease caused by *Phaeomoniella* spp. and *Phaeoacremonium* spp. (Fourie and Halleen. 2001, Winelands, 12, 19-23; 2002. Aus. Plant Path. 31:425-426.). Black foot disease and Petri disease primarily infect grapevine propagation material and newly planted vines and are either individually or collectively responsible for the decline of young vines, reduction or loss in productivity and young vine death. Older vines that have been infected with these diseases show a stunting phenotype and low or even no fruit carrying potential. As a result, grape farmers are forced to replant young infected vineyards at a substantial cost and loss of production. The problem, however, often arises at the nurseries that supply the propagation material. Research has shown that the primary source of infected material is mother block material and nurseries, with less than 50% of propagation material yielding healthy saleable plants (Fourie and Halleen, 2004, Aus. Plant Path. 33: 313-315; Halleen et al., 2003. Aus. Plant Path.32: 47-52). Halleen et al. (2007, Plant Path. 56, 637-645) evaluated 13 fungicides, representing 10 different chemical classes for in vitro mycelial inhibition of *Cylindrocarpon* and *Campylocarpon* spp, the causal agents of black foot disease. Only prochloraz manganese chloride, imazalil and benomyl were able to effectively reduce mycelial growth in all fungal strains tested, but failed to protect propagation against black foot disease under field conditions. This study showed that most of the chemical treatments were ineffective and inconsistent in protecting grapevine against black foot disease.

Plant tissue culture is an important technique in the grapevine and fruit industry for the supply of virus- and pathogen-free planting material for the establishment of new vineyards and orchards. A second tissue culture technique, namely embryo rescue, plays an important role in the breeding of new cultivars, especially seedless grape varieties. Although these techniques have been well established in supplying good quality planting material there are still a number of aspects that influence the success rate of establishing field material, into an in vitro environment. One of the most important factors is explant quality, which can be greatly affected by the microbial population (fungi, bacteria and yeasts) present on field plant material (Cassells, 2001, Proc. IV IS on In Vitro Cult. & Hort. Breeding, Eds. S. Sorvari et al., 353-350). The rich medium used to establish explant material in vitro is also a perfect medium for the growth of these microbes, making the sterilization strategy which is employed a crucial step in the successful establishment of explant material from the field back into in vitro culture (Cassells, 2001, Proc. IV IS on In Vitro Cult. & Hort. Breeding, Eds. S. Sorvari et al., 353-350). Sterilization of explant material usually involves a combination of hot water treatment, alcohol and NaOCl₂ treatments (Cassells, 2000, Plant Cell Biology (Ed. Spier, R.E.), Wiley, Chichester, 577-586), but this only removes some of the surface microbes, while endophytic bacteria and fungi survive, leading to contamination when the explant is placed on the culture medium. Tissue culture laboratories rely on antibiotics to control both fungal and bacterial infections, but it is rarely the case that a single microbe is present on the explant material, requiring combinations of antibiotics, which can lead to phytotoxicity (Estopá, 2001, Plant Cell, Tissue & Organ Culture 65, 211-220). PPMTM, containing methylchloroisothiazolinone/methylisothiazolinone as active ingredients, is currently the only chemical product on the market that claims to inhibit the growth of both bacterial and fungal pathogens, but it has been reported to interfere with plant development, especially in grapevine tissue culture (Compton and Koch, 2001, In Vitro Cell Dev. Biol. Plant 37, 259-261).

Alternatives to chemical microbicides, such as bio-control agents, have given producers an environmentally friendly means of protecting their produce, but with an ever-changing environment, the efficacy of biological control is unpredictable and cannot guarantee protection against microbial pathogens.

With the existing antimicrobial agents (mostly chemical microbicides) losing potency towards many of the pathogens due to resistance, increased global opposition to the use of chemical control and the movement towards more natural and organic farming practices, there is a need for natural bio-control agents with a broad spectrum of activity that can ensure the establishment of high quality planting material and also protect produce during post-harvest storage.

Isolated bioactive antibiotic peptides from Bacillus brevis and Bacillus polymyxa have been shown to be active against Botrytis grey mould in strawberries (Haggaga, 2008, Archives of Phytopathology and Plant Protection 41(7), 477-491).

### SUMMARY OF THE INVENTION

According to a first embodiment of the invention, there is provided a method of preventing or controlling microbial growth on plants, plant material, plant parts or in media used to sustain plants or plant material, the method comprising the step of applying an antimicrobial composition to the plants, plant material, plant parts or media,
wherein the antimicrobial composition comprises a cyclic decapeptide produced from *Bacillus aneurinolyticus* as an active agent, the cyclic decapeptide being a tyrocidine, tryptocidine or phenycidine or an analogue thereof comprising an amino acid sequence of cyclo(valine-X₁-leucine-D-phenylalanine-proline-X₂X₃-X₄-X₅-X₆) (SEQ ID NO: 1), where:

- X₁: is ornithine or lysine;
- X₂: is valine, leucine, isoleucine, phenylalanine, tryptophan or tyrosine;

- X₃: is the D-isomer of valine, leucine, isoleucine, phenylalanine, tryptophan or tyrosine;
- X₄: is asparagine or glutamine;
- X₅: is glutamine or asparagine; and
- X₆: is tyrosine, phenylalanine or tryptophan.

The cyclic decapeptide may have an amino acid selected from any one of SEQ ID NOS: 6-21, SEQ ID NOS: 22-37 or SEQ ID NOS: 38-59, any one of SEQ ID NOS: 60-75, SEQ ID NOS: 76-91 or SEQ ID NOS: 92-113, or any one of SEQ ID NOS: 114-129, SEQ ID NOS: 130-145, SEQ ID NOS: 146-161 or SEQ ID NOS: 162-167.

The composition may contain two or more of the cyclic decapeptides described above.

The composition may also contain an organism which naturally produces the cyclic decapeptide(s).

The composition may be an antifungal composition for controlling the growth of one or more of the following fungi: *Phaeoacremonium* spp. (such as *Phaeoacremonium aleophilum), Phomopsis viticola, Fusarium* spp. (such as *Fusarium solani, F. oxysporum, F. verticilliodes), Cylindrocarpon* spp. (such as *Cilidocarpon liriodendra), Botrytis cinerea, Talaromyces* spp., *Aspergillus* spp. *Penicillium* spp., *Monilinia* spp., *Trichoderma* spp., or *Phaeomoniella* spp..

The composition may be an antibacterial composition.

The composition may be capable of treating or preventing blue mould, brown rot, green mold, vine wilt and root rot, black foot disease, young grapevine decline or Petri disease.

The cyclic decapeptides or mixture of cyclic peptides or analogues/derivative(s) thereof may be chemically modified to improve solubility, bio-availability and/or bio-activity and/or to limit toxicity. Modification methods may include oxidation, hydroxylation, acylation, amidation, coupling of an organic moiety, hydroxyl, carbonyl, carboxyl, amino, methyl or sugar/sugar derivative group substitution and biosynthetic modification.

The composition may be suitably formulated to improve activity, stability and/or bio-availability and/or to limit toxicity. Formulations may contain biological salts, lipids or lipid derivatives, polysaccharides or polysaccharide derivatives, sugars or sugar derivatives, bio-friendly or approved GRAS additives.

The composition may also include one or more other antimicrobial or antifungal compounds, including natural peptides, lipopeptides or antibiotics from animal, microbial or plant origin or chemically produced biocides or antibiotics.

The composition may include a combination of one or more tyrocidines and gramicidin S or derivatives thereof.

The plants may be embryonic, young or mature plants, and may be fruit-producing plants, vegetable-producing plants, row crops, vegetable crops, ornamental plants, grasses or trees. For example, the plants can be grapevines, strawberry plants, stone fruit trees (e.g. plums, cherries, peaches, nectarines, apricots and almonds), apple trees, pear trees, cereals such as wheat and the like.

The plant material or plant parts may be plant cuttings, roots, plant material for cell culture, explants, flowers, fruits, vegetables, seeds or cereals. The plant material may be dried plant material, such as is found in dry animal feed. The fruits may be fruits which are susceptible to *B*. *cinerea* and other spoilage pathogens, infection and spoilage, such as grapes and strawberries, various berries or cherries and tree fruits like figs, pears, apples, peaches, apricots or plums. Vegetables include all varieties growing above and underground.

According to a second embodiment of the invention, there is provided a method of improving plant growth or plant vigour or prolonging the life of cut flowers, the method comprising the step of applying the composition described above to the plants, to plant material, plant parts or growth media, or to vase water for the cut flowers.

### BRIEF DESCRIPTION OF THE FIGURES

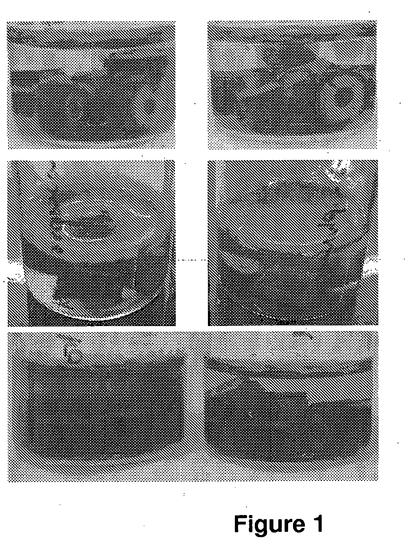

- **Figure 1:**: Fungal growth development after 5 days of incubation in the absence (controls, left panels) and presence of gramicidin S (treatment, panels on right) with **A** Cylindrocarpon (20 mg/L gramicidin S), **B** Phomopsis (10 mg/L gramicidin S), **C** Phaeoacremonium (20 mg/L gramicidin S).

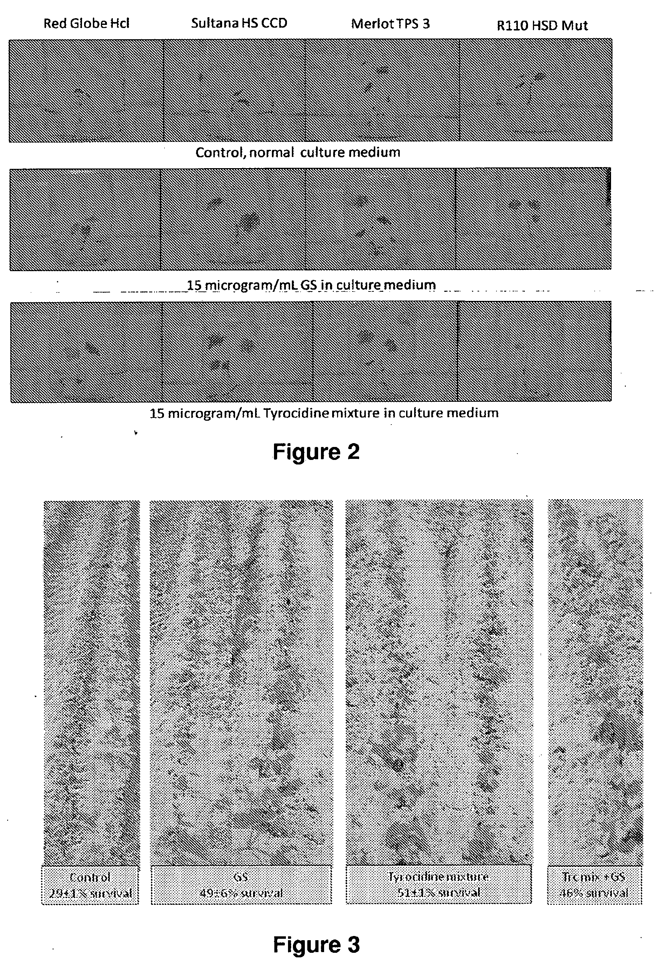

- **Figure 2:**: The effect of gramicidin S (GS) and a tyrocidine (Trc) mixture at 15 mg/L on the development of a selection of grapevine tissue culture plants.
- **Figure 3:**: The effect of gramicidin S (GS), a tyrocidine mixture (Tyr) and a tyrocidine/gramicidin S mixture at 30 mg/L used for grape stickling sterilization on the development of grapevine plants.

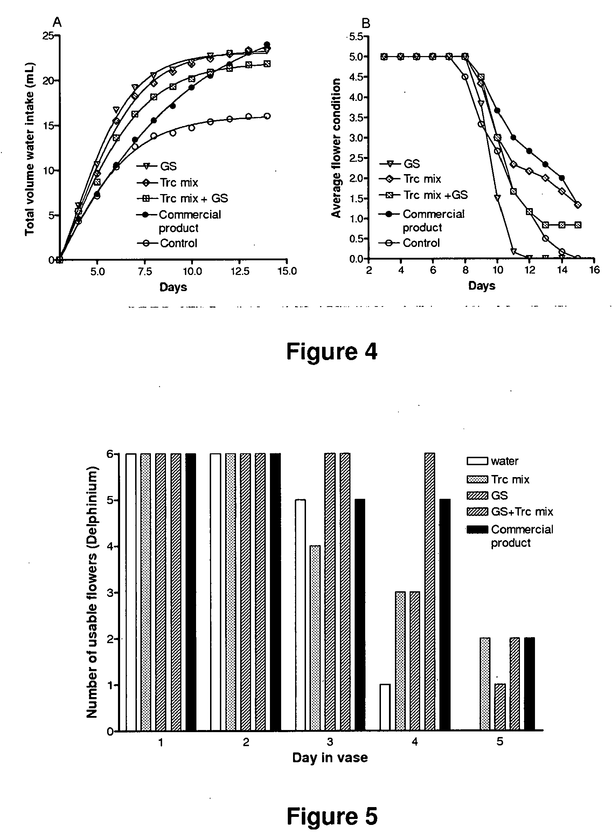

- **Figure 4:**: Effect of vase water treatment on the water uptake of mini African daisies (*Gerbera* ssp.) (A) and the flower condition (B) over 15 days. Controls contained only tap water, those with commercial product received the dosage as specified by supplier and trial flowers received a 35 mg/L tyrocidine mixture, 35 mg/L gramicidin S (GS) or a gramicidin S and tyrocidine mixture (containing 35 mg/L of each).
- **Figure 5:**: Effect of vase water treatment on the vase life of Delphinium flowers over 5 days, with a flower condition of 4 or 5 deemed as usable flowers. Controls contained only tap water, those with commercial product received the dosage as specified by the supplier and the peptide trial contained a 25 mg/L tyrocidine mixture, 25 mg/L gramicidin S (GS) or a gramicidin S and tyrocidine mixture (containing 25 mg/L of each).

### DETAILED DESCRIPTION OF THE INVENTION

Methods for controlling or preventing the growth of microbial pathogens, and in particular fungal pathogens, on plants, plant parts and plant material are described herein. The active agents used to control these pathogens are tyrocidines, tryptocidines, phenycidines or analogues thereof.

The tyrocidines, tryptocidines, phenycidines and gramicidin S are a family of natural β-sheet cyclic decapeptides which adopt similar backbone conformations/molecular topologies, are highly conserved and have high sequence identity. They are produced by *Bacillus* and *Brevibacillus* spp.

Tyrocidines and gramicidin S contain the pentapeptide sequence Val-Orn-Leu-D-Phe-Pro (SEQ ID NO: 4). This pentapeptide moiety can vary in its cationic residue, with the ornithine residue being replaceable by lysine for both tyrocidines and gramicidin S, and the valine or leucine residues also being replaceable by leucine, isoleucine or valine for the tyrocidines.

Gramicidin S has a highly conserved sequence which is a repeat of the pentapeptide unit described above, namely *cyclo*(Val-Orn/Lys-Leu-D-Phe-Pro)₂ (SEQ ID NO: 3). Instead of the pentapetide unit being repeated, tyrocidines comprise a variable pentapeptide moiety of Phe-D-Phe-Asn-Gln-Tyr (SEQ ID NO: 5), or a derivative thereof where the three aromatic residues are independently replaceable by tryptophan, phenylalanine or tyrosine.

The primary chemical structures of gramicidin S and one of the tyrocidines (tyrocidine A) are shown below:

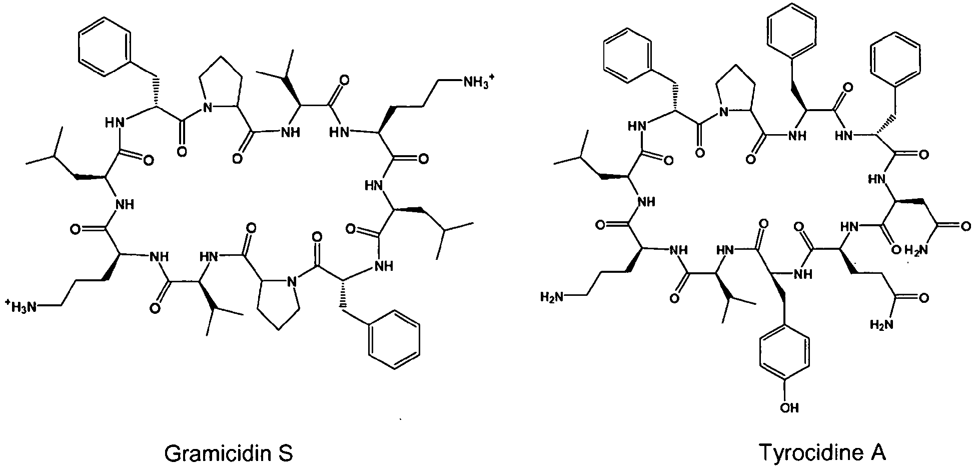

Studies on tyrocidines have shown that they are active against *Neurospora crassa* (Mach and Slayman 1966, BBA 124, 351-36; Trevillyan and Pall, 1979, J. Bact. 397-403) and that the tyrocidine-gramicidin complex (gramicidins are linear neutral 15-mer peptides not related to gramicidin S), tyrothricin, is active against *Candica albicans* (Kretschmar et al., 1996, Mycoses. 39, 45-50). Tyrothricin was the first antibiotic to be used in clinical practices, but later fell into disrepute due to its haemolytic toxicity (Dubos and Cattaneo, 1939, J. Exp. Med. 70, 249; Hotchkiss and Dubos, 1941, J. Biol. Chem. 141, 155; Bradshaw, 2003 BioDrugs 17, 233-240). The applicant is, however, not aware of any studies which have been conducted on tyrothricin producers (e.g. *Bacillus aneurinolyticus,* commonly referred to as the Dubos strain of *Bacillus brevis)* or tyrocidines in the bio-control of plant pathogens. This is possibly due to the perceived high toxicity of these peptides, although it would appear that this perception is unfounded (Rautenbach et al. 2007, BBA Biomembr. 1768, 1488-1497). The tyrothricin complex has also been used in throat lozenges (1 mg tyrothricin per lozenge) under the trade name Tyrozets, indicating its relative safety for human consumption, but this has since been discontinued due to questionable efficacy.

The cyclic decapeptides of the present invention are known tyrocidines, tryptocidines, phenycidines or anlaogues therof, which have a highly conserved amino acid sequence comprising Val-X₁-Leu-D-Phe-Pro-X₂-X₃-X₄-X₅-X₆ (SEQ ID NO: 2), or a derivative or analogue thereof, where X₁ is ornithine or lysine.

Suitable analogues of the tyrocidines, tryptocidines or phenycidines may be those including one or more of the following substitutions:
the valine residue substituted with a leucine or isoleucine residue or hydrophobic amino acid or analogue/derivative;
the leucine residue substituted with an isoleucine or valine residue or hydrophobic amino acid or analogue/derivative;
the proline residue being replaced by hydroxyproline residue or analogue/derivative thereof;
the phenylalanine residue substituted with a tryptophan or tyrosine residue or aromatic analogue/derivative thereof;
the ornithine residue substituted with a lysine or a cationic amino acid or an analogue/derivative thereof;
X₂ being valine, leucine, isoleucine, phenylalanine, tryptophan or tyrosine or a hydrophobic amino acid or analogue/derivative;
X₃ being a D-isomer of valine, leucine, isoleucine, phenylalanine, tryptophan or tyrosine or a hydrophobic amino acid or analogue/derivative thereof; or alternatively being an ornithine, lysine or cationic amino acid or analogue/derivative thereof;
X₄ being asparagine, glutamine or leucine or an analogue or derivative thereof;
X₅ being glutamine or a polar amino acid or analogue/derivative thereof; or alternatively being the D-isomer of valine, leucine, isoleucine, or a hydrophobic amino acid or analogue/derivative thereof; and
X₆ being a tyrosine, phenylalanine or tryptophan or proline residue.

More preferably, the cyclic decapeptide derivatives may be one or more of the peptides selected from the group consisting of:
Tyrocidine analogues:
   *Cyclo*-(VKLfPWwNOY) (Tyrocidine C₁) (SEQ ID NO: 6)
      *Cyclo*-(VOLfPWwNQY) (Tyrocidine C) (SEQ ID NO: 7)
      *Cyclo*-(VKLfPWfNQY) (Tyrocidine B₁) (SEQ ID NO: 8)
      *Cyclo*-(VOLfPWfNQY) (Tyrocidine B) (SEQ ID NO: 9)
      *Cyclo*-(VKLfPFwNQY) (Tyrocidine B₁) (SEQ ID NO: 10)
      *Cyclo*-(VOLfPFwNQY) (Tyrocidine B') (SEQ ID NO: 11)
      *Cyclo*-(VKLfPFfNQY) (Tyrocidine A₁) (SEQ ID NO: 12)
      *Cyclo*-(VOLfPFfNQY) (Tyrocidine A) (SEQ ID NO: 12)
      *Cyclo*-(VKLfPYwNQY) (SEQ ID NO: 14)
      *Cyclo*-(VOLfPYwNQY) (SEQ ID NO: 15)
      *Cyc*/*o*-(VKLfPYfNQY) (SEQ ID NO: 16)
      *Cyc*/*o*-(VOLfPYfNQY) (SEQ ID NO: 17)
      *Cyc*/*o*-(VKLfPFyNQY) (SEQ ID NO: 18)
      *Cyc*/*o*-(VOLfPFyNQY) (SEQ ID NO: 19)
      *Cyclo*-(VKLfPWyNQY) (SEQ ID NO: 20)
      *Cyclo*-(VOLfPWyNQY) (SEQ ID NO: 21)
   *Cyclo*-(LKLfPWwNQY) (SEQ ID NO: 22)
      *Cyc*/*o*-(LOLfPWwNQY) (SEQ ID NO: 23)
      *Cyc*/*o*-(LKLfPWfNQY) (SEQ ID NO: 24)
      *Cyc*/*o*-(LOLfPWfNQY) (SEQ ID NO: 25)
      *Cyc*/*o*-(LKLfPFwNQY) (SEQ ID NO: 26)
      *Cyc*/*o*-(LOLfPFwNQY) (SEQ ID NO: 27)
   *Cyclo*-(LKLfPFfNQY) (SEQ ID NO: 28)
      *Cyclo*-(LOLfPFfNQY) (SEQ ID NO: 29)
      *Cyclo*-(LKLfPYwNQY) (SEQ ID NO: 30)
      *Cyclo*-(LOLfPYwNQY) (SEQ ID NO: 31)
      *Cyclo*-(LKLfPYfNQY) (SEQ ID NO: 32)
      *Cyclo*-(LOLfPYfNQY) (SEQ ID NO: 33)
      *Cyclo*-(LKLfPFyNQY) (SEQ ID NO: 34)
      *Cyclo*-(LOLfPFyNQY) (SEQ ID NO: 35)
      *Cyclo*-(LKLfPWyNQY) (SEQ ID NO: 36)
      *Cyclo*-(LOLfPWyNQY) (SEQ ID NO: 37)
   *Cyclo*-(IKLfPWwNQY) (SEQ ID NO: 38)
      *Cyclo*-(IOLfPWwNQY) (SEQ ID NO: 39)
      *Cyclo*-(IKLfPWfNQY) (SEQ ID NO: 40)
      *Cyclo*-(IOLfPWfNQY) (SEQ ID NO: 41)
      *Cyclo*-(IKLfPFwNQY) (SEQ ID NO: 42)
      *Cyclo*-(IOLfPFwNQY) (SEQ ID NO: 43)
      *Cyclo*-(IKLfPFfNQY) (SEQ ID NO: 44)
      *Cyclo*-(IOLfPFfNQY) (SEQ ID NO: 45)
      *Cyclo*-(IKLfPYwNQY) (SEQ ID NO: 46)
      *Cyclo*-(IOLfPYwNQY) (SEQ ID NO: 47)
      *Cyclo*-(IKLfPYfNQY) (SEQ ID NO: 48)
      *Cyclo*-(IOLfPYfNCQY) (SEQ ID NO: 49)
      *Cyclo*-(IKLfPFyNQY) (SEQ ID NO: 50)
      *Cyclo*-(IOLfPFyNQY) (SEQ ID NO: 51)
      *Cyclo*-(IKLfPWyNQY) (SEQ ID NO: 52)
      *Cyclo*-(IOLfPWyNQY) (SEQ ID NO: 53)
      *Cyclo*-(VKLfPLwNQY) (SEQ ID NO: 54)
      *Cyclo*-(VOLfPLwNQY) (SEQ ID NO: 55)
      *Cyclo*-(VKLfPLfNQY) (SEQ ID NO: 56)
      *Cyclo*-(VOLfPLfNQY) (SEQ ID NO: 57)
      *Cyclo*-(VKLfPLyNQY) (SEQ ID NO: 58)
      *Cyclo*-(VOLfPLyNQY) (SEQ ID NO: 59)
Tryptocidine analogues:
   *Cyclo*-(VKLfPWwNQW) (SEQ ID NO: 60)
      *Cyclo*-(VOLfPWwNQW) (Tryptocidine C) (SEQ ID NO: 61)
      *Cyclo*-(VKLfPWfNQW) (SEQ ID NO: 62)
      *Cyclo*-(VOLfPWfNQW) (Tryptocidine B) (SEQ ID NO: 63)
      *Cyclo*-(VKLfPFwNQW) (SEQ ID NO: 64)
      *Cyclo*-(VOLfPFwNQW) (SEQ ID NO: 65)
      *Cyclo*-(VKLfPFfNQW) (SEQ ID NO: 66)
      *Cyclo*-(VOLfPFfNQW) (Tryptocidine A) (SEQ ID NO: 67)
      *Cyclo*-(VKLfPYwNQW) (SEQ ID NO: 68)
      *Cyclo*-(VOLfPYwNQW) (SEQ ID NO: 69)
      *Cyclo*-(VKLfPYfNQW) (SEQ ID NO: 70)
      *Cyclo*-(VOLfPYfNQW) (SEQ ID NO: 71)
      *Cyclo*-(VKLfPFyNQW) (SEQ ID NO: 72)
      *Cyclo*-(VOLfPFyNQW) (SEQ ID NO: 73)
      *Cyclo*-(VKLfPWyNQW) (SEQ ID NO: 74)
      *Cyclo*-(VOLfPWyNQW) (SEQ ID NO: 75)
   *Cyclo*-(LKLfPWwNQW) (SEQ ID NO: 76)
      *Cyclo*-(LOLfPWwNQW) (SEQ ID NO: 77)
      *Cyclo*-(LKLfPWfNQW) (SEQ ID NO: 78)
      *Cyclo*-(LOLfPWfNQW) (SEQ ID NO: 79)
      *Cyclo*-(LKLfPFwNQW) (SEQ ID NO: 80)
      *Cyclo*-(LOLfPFwNQW) (SEQ ID NO: 81)
      *Cyclo*-(LKLfPFfNQW) (SEQ ID NO: 82)
      *Cyclo*-(LOLfPFfNQW) (SEQ ID NO: 83)
      *Cyclo*-(LKLfPYwNQW) (SEQ ID NO: 84)
      *Cyclo*-(LOLfPYwNQW) (SEQ ID NO: 85)
      *Cyclo*-(LKLfPYfNQW) (SEQ ID NO: 86)
      *Cyclo*-(LOLfPYfNQW) (SEQ ID NO: 87)
      *Cyclo*-(LKLfPFyNQW) (SEQ ID NO: 88)
      *Cyclo*-(LOLfPFyNQW) (SEQ ID NO: 89)
      *Cyclo*-(LKLfPWyNQW) (SEQ ID NO: 90)
      *Cyclo*-(LOLfPWyNQW) (SEQ ID NO: 91)
   *Cyclo*-(IKLfPWwNQW) (SEQ ID NO: 92)
      *Cyclo*-(IOLfPWwNQW) (SEQ ID NO: 93)
      *Cyclo*-(IKLfP(Wf)NQW) (SEQ ID NO: 94)
      *Cyclo*-(IOLfP(Wf)NQW) (SEQ ID NO: 95)
      *Cyclo*-(IKLfP(Fw)NQW) (SEQ ID NO: 96)
      *Cyclo*-(IOLfP(Fw)NQW) (SEQ ID NO: 97)
      Cyc/o-(IKLfPFfNQW) (SEQ ID NO: 98)
      *Cyclo*-(IOLfPFfNQW) (SEQ ID NO: 99)
      *Cyclo*-(IKLfPYwNQW) (SEQ ID NO: 100)
      *Cyclo*-(IOLfPYwNQW) (SEQ ID NO: 101)
      *Cyclo*-(IKLfPYfNQW) (SEQ ID NO: 102)
      *Cyclo*-(IOLfPYfNQW) (SEQ ID NO: 103)
      *Cyclo*-(IKLfPFyNQW) (SEQ ID NO: 104)
      *Cyclo*-(IOLfPFyNQW) (SEQ ID NO: 105)
      *Cyclo*-(IKLfPWyNQW) (SEQ ID NO: 106)
      *Cyclo*-(IOLfPWyNQW) (SEQ ID NO: 107)
   *Cyclo*-(VKLfPLwNQW) (SEQ ID NO: 108)
      *Cyclo*-(VOLfPLwNQW) (SEQ ID NO: 109)
      *Cyclo*-(VKLfPLfNQW) (SEQ ID NO: 110)
      *Cyclo*-(VOLfPLfNQW) (SEQ ID NO: 111)
      *Cyclo*-(VKLfPLyNQW) (SEQ ID NO: 112)
      *Cyclo*-(VOLfPLyNQW) (SEQ ID NO: 113)
Phenycidine analogues:
   *Cyclo*-(VKLfPWwNQF) (SEQ ID NO: 114)
      *Cyclo*-(VOLfPWwNQF) (Phenycidine C) (SEQ ID NO: 115)
      *Cyclo*-(VKLfPWfNQF) (SEQ ID NO: 116)
      *Cyclo*-(VOLfPWfNQF) (Phenycidine B) (SEQ ID NO: 117)
      *Cyclo*-(VKLfPFwNQF) (SEQ ID NO: 118)
      *Cyclo*-(VOLfPFwNQF) (SEQ ID NO: 119)
      *Cyclo*-(VKLfPFfNQF) (SEQ ID NO: 120)
      *Cyclo*-(VOLfPFfNQF) (Phenycidine A or Tyrocidine E) (SEQ ID NO: 121)
      *Cyclo*-(VKLfPYwNQF) (SEQ ID NO: 122)
      *Cyclo*-(VOLfPYwNQF) (SEQ ID NO: 123)
      *Cyclo*-(VKLfPYfNQF) (SEQ ID NO: 124)
      *Cyclo*-(VOLfPYfNQF) (SEQ ID NO: 125)
      *Cyclo*-(VKLfPFyNQF) (SEQ ID NO: 126)
      *Cyclo*-(VOLfPFyNQF) (SEQ ID NO: 127)
      *Cyclo*-(VKLfPWyNQF) (SEQ ID NO: 128)
      *Cyclo*-(VOLfPWyNQF) (SEQ ID NO: 129)
   *Cyclo*-(LKLfPWwNQF) (SEQ ID NO: 130)
      *Cyclo*-(LOLfPWwNQF) (SEQ ID NO: 131)
      *Cyclo*-(LKLfPWfNQF) (SEQ ID NO: 132)
      *Cyclo*-(LOLfPWfNQF) (SEQ ID NO: 133)
      *Cyclo*-(LKLfPFwNQF) (SEQ ID NO: 134)
      *Cyclo*-(LOLfPFwNQF) (SEQ ID NO: 135)
      *Cyclo*-(LKLfPYwNQF) (SEQ ID NO: 136)
      *Cyclo*-(LOLfPYwNQF) (SEQ ID NO: 137)
      *Cyclo*-(LKLfPYfNQF) (SEQ ID NO: 138)
      *Cyclo*-(LOLfPYfNQF) (SEQ ID NO: 139)
      *Cyclo*-(LKLfPFyNQF) (SEQ ID NO: 140)
      *Cyclo*-(LOLfPFyNQF) (SEQ ID NO: 141)
      *Cyclo*-(LKLfPWyNQF) (SEQ ID NO: 142)
      *Cyclo*-(LOLfPWyNQF) (SEQ ID NO: 143)
      *Cyclo*-(LKLfPFfNQF) (SEQ ID NO: 144)
      *Cyclo*-(LOLfPFfNQF) (SEQ ID NO: 145)
   *Cyclo*-(IKLfPWwNQF) (SEQ ID NO: 146)
      *Cyclo*-(IOLfPWwNQF) (SEQ ID NO: 147)
      *Cyclo*-(IKLfPWfNQF) (SEQ ID NO: 148)
      *Cyclo*-(IOLfPWfNQF) (SEQ ID NO: 149)
      *Cyclo*-(IKLfPFwNQF) (SEQ ID NO: 150)
      *Cyclo*-(IOLfPFwNQF) (SEQ ID NO: 151)
      *Cyclo*-(IKLfPYwNQF) (SEQ ID NO: 152)
      *Cyclo*-(IOLfPYwNQF) (SEQ ID NO: 153)
      *Cyclo*-(IKLfPYfNQF) (SEQ ID NO: 154)
      *Cyclo*-(IOLfPYfNQF) (SEQ ID NO: 155)
      *Cyclo*-(IKLfPFyNQF) (SEQ ID NO: 156)
      *Cyclo*-(IOLfPFyNQF) (SEQ ID NO: 157)
      *Cyclo*-(IKLfPWyNQF) (SEQ ID NO: 158)
      *Cyclo*-(IOLfPWyNQF) (SEQ ID NO: 159)
      *Cyclo*-(IKLfPFfNQF) (SEQ ID NO: 160)
      *Cyclo*-(IOLfPFfNQF) (SEQ ID NO: 161)
      *Cyclo*-(VKLfPLwNQF) (SEQ ID NO: 162)
      *Cyclo*-(VOLfPLwNQF) (SEQ ID NO: 163)
      *Cyclo*-(VKLfPLfNQF) (SEQ ID NO: 164)
      *Cyclo*-(VOLfPLfNQF) (SEQ ID NO: 165)
      *Cyclo*-(VKLfPLyNQF) (SEQ ID NO: 166)
      *Cyclo*-(VOLfPLyNQF) (SEQ ID NO: 167)
where standard upper case abbreviations denote L-amino acids, with the exception of O for ornithine, lower case abbreviations denote a D-residue, and *Cyclo* indicates amino to carboxy-terminal cyclisation via an amide bond.

References hereafter to "cyclic decapeptides" refer to the sequences stated above and analogues or derivatives thereof.

The applicant has found that tyrocidines and derivatives thereof, and compositions containing these or their producers, can be used as bio-control agents to control or prevent the growth of antimicrobial pathogens on plants or plant material or culturing media and environments. For example, they can be used:
(1) to prevent latent fungal pathogen carry-over into nursery propagated plants and promote growth and survival;
(2) to control and prevent fungal infections in embryonic and young plants and promote growth and survival;
(3) to control and prevent fungal and microbial infections in plant material and media for use in plant cell cultures;
(4) to afford protection against post-harvest fungal pathogens infecting harvested fruits;
(5) to improve cut-flower preservation by improving water uptake and preventing/limiting flower stem infections.

The plants can be fruit-producing plants, vegetable-producing plants, row crops, vegetable crops, ornamental plants, grasses or trees. For example, the plants can be berry carrying plants such as grapevines, strawberry plants, apple trees, pear trees, stone fruit trees, cereals such as wheat and the like.

Plant material includes plant cuttings, roots, explants, plant material for cell culture, flowers, fruits (such as strawberries, grapes, cherries, apples, pears, peaches and the like), seeds or cereals.

The cyclic decapeptides can be produced by their natural bacterial producers, by genetically modifying a suitable microorganism or by using an organic/semi-synthetic system. The amino acid residues in the derivatives or analogues can separately or in combination be replaced in the core cyclic decapeptide sequence (cyclo(valine-ornithine-leucine-D-phenylalanine-proline-X₂-X₃-X₄-X₅-X₆) (SEQ ID NO: 1) by bacterial/microbial production or using organic/semisynthetic systems.

The cyclic decapeptides can be chemically modified to improve solubility, bio-availability and/or bio-activity and/or to limit toxicity. Modification methods include oxidation, hydroxylation, acylation, amidation, coupling of an organic moiety, hydroxyl, carbonyl, carboxyl, amino, methyl or sugar/sugar derivative group substitution and biosynthetic modification.

The cyclic decapeptides, mixtures thereof or modifications thereof can be formulated into a suitable composition for use on plants, plant parts or plant material, flower preservation, in culturing media, in culturing facilities or nursery environments, or on plant propagation equipment. The composition can be suitably formulated to improve activity, stability and/or bio-availability and/or to limit toxicity. Formulations can contain biological salts, lipids or lipid derivatives, polysaccharides or polysaccharide derivatives, sugars or sugar derivatives, bio-friendly or approved GRAS additives.

The composition can be formulated in various types of formulations, such as solutions, wettable powders, soluble powders, tablets and water-soluble or dispersible granules. The composition can also be formulated as a concentrated stock (which is diluted in an aqueous solution prior to conventional spray application) or as a ready to use product.

A surfactant can be used as a wetting, solubilizing and penetrating agent. Suitable surfactants include peptide derived surfactants (i.e. surfactin and iturin), non-ionic surfactants, anionic surfactants and amphoteric surfactants, such as cholic acids, alkyl sulfate salts, alkylsulfonic acid salts, alkylarylsulfonic acid salts, alkyl aryl ethers and their polyoxyethylene derivatives, polyethylene glycol ethers, polyol esters and sugar alcohol derivatives.

Other components of the formulation can include additional surface active agents, solvents, cosolvents, dyes, U.V. (ultra-violet) protectants, antioxidants, antifoams, stickers, spreaders, anti-foaming agents, preservatives, humectants, buffers, carriers, emulsifiers, wetting agents, dispersants, fixing agents, disintegrators, acid solubilisers or other components which facilitate product handling and application. These carriers, diluents, auxiliary agents and so forth are preferably selected to optimize the antifungal action on plants or plant material.

Solid carriers can include, for example, the following materials in fine powder or granular form: agarose/agar containing cell culture media or dried cell culture media; organic-type fertilisers; clays (e.g. kaolinite, diatomaceous earth, synthetic hydrated silicon oxide, Fubasami clay, bentonite, acid clay); talc and other inorganic minerals (e.g. sericite, quartz powder, sulfur powder, activated carbon, calcium carbonate); and chemical fertilizers (e.g. ammonium sulfate, ammonium phosphate, ammonium nitrate, ammonium chloride, urea). Liquid carriers can include, for example, cell culture media, water; alcohols (e.g. methanol, ethanol, isopropanol); ketones (e.g. acetone, methyl ethyl ketone, cyclohexanone); esters (e.g. ethyl acetate, butyl acetate); nitriles (e.g. acetonitrile, isobutyronitrile); and acid amides (e.g. dimethylformamide, dimethylacetamide), as well as dilute bases (e.g. sodium hydroxide, potassium hydroxide and amines)

Other auxiliary agents can include, for example, adhesive agents and dispersing agents, such as casein, gelatin, polysaccharides (e.g. powdered starch, gum arabic, cellulose derivatives, alginic acid, chitin), lignin derivatives and synthetic water-soluble polymers (e.g. polyvinyl alcohol, polyvinyl pyrrolidone, polyacrylic acid); salts (eg. citrate, chloride, sulphate, acetate, ammonium, bicarbonate, phosphate salts and like) and stabilizers such as PAP (isopropyl acid phosphate), BHT (2,6-di-tert-butyl-4-methylphenol), BHA (2-/3-tert-butyl-4-methyoxyphenol), vegetable oils, mineral oils, phospholipids, waxes, fatty acids and fatty acid esters.

Conventional plant growth regulators, herbicides, fungicides, bactericides, insecticides, nematicides, acaricides, biochemical pesticides, plant produced pesticides (botanicals), cell culture media components or plant nutrients and so forth can also be incorporated into the composition of the present invention.

The composition can also include one or more other antimicrobial or antifungal compounds, including natural peptides, lipopeptides or antibiotics from animal, microbial or plant origin or chemically produced fungicides or antibiotics. For example, the composition can include a tyrocidine-gramicidin (termed tyrothricin) complex.

The composition may be diluted in water, water organic mixture or with liquid carrier and sprayed or applied in controlled environments on the plant or plant material to be treated or used to wash plant materials or environment/systems/equipment or mixed with cell culture media or plant propagation media. Alternatively, the composition may be directly applied to the soil (in which the plant will be grown or is growing) with or without granular fertilizers or organic-type fertilisers for propagation of cultured plants or the improved growth of plants.

Tyrocidines, tryptocidines and phenycidines and derivatives/analogues thereof are shown below to be broad spectrum microbicides that are active against both bacterial and fungal contamination. The cyclic decapeptides analogous to the tyrocidines also show low phytotoxicity. All the cyclic decapeptides are heat stable, work over a wide pH range and are relatively insensitive to mineral salts. Moreover, because they are cyclic peptides, they are less likely to be rapidly degraded like linear peptides and are therefore suitable for offering long term protection possibilities.

The invention will now be described in more detail by way of the following non-limiting examples.

### Examples

### Antimicrobial activity

The antimicrobial activities of a tyrocidine mixture (Trc mix), as well as purified tyrocidines, tryptocidine C, phenycidine A and gramicidin S (GS) were analyzed. The Trc mix typically contained TrcA/A₁ (30-35%), TrcB/B₁ (30-35%), TrcC/C₁ (20-25%), TpcA/B/C (5-10%) and PhcA and minor analogues (<5%). GS used in the *in vivo* trials typically contained gramicidin S (>90%) and gramicidin S analogues (<10%).

Microdilution broth and agar assays were used to assess the antifungal activity of the cyclic decapeptides in liquid media and on solid (gel) media, respectively. In broth and agar medium, the Trc mix displayed significant activity against selected fungal pathogens, for example *Phaeoacremonium aleophilum, Phomopsis viticola, Fusarium solani, F. oxysporum, F. verticilliodes, Cifidocarpon liriodendri* and *Botrytis cinerea* (Table 1). GS also showed significant activity against these fungal pathogens. The Trc mix and GS showed a broad spectrum low micromolar antibacterial activity, in particular against the food pathogen *L. monocytogenes.*

**Table 1: Antifungal activity of the natural tyrocidine mixture and gramicidin S against selected fungal and bacterial target organisms.**

| **Microbial target** | Trc mix | | GS | |
|---|---|---|---|---|
| | MIC+ SEM | | MIC+SEM | |
| **Fungal species** | Broth | Agar | Broth | Agar |
| *Fusarium solani* | 9.3 ± 1.2 | > 100 | 2.5 ± 0.1 | 5.5 ± 1.3 |
| *Fusarium oxysporum* | 10 ± 1.1 | nd | 2.6 ± 0.2 | nd |
| *Fusarium verticilliodes* | 12 ± 2.9 | nd | 3.5 ± 0.3 | nd |
| *Botrytis cinerea* | 4.8 ± 0.7 | 7.6 ± 1.7 | 2.8 ± 0.3 | 2.5 ± 0.3 |
| *Cylindrocarpon liriodendri* | 2.9 ± 0.1 | <10 | 2.3 ± 0.1 | <10 |
| *Aspergillus fumigatus ATCC 204305* | 8.6 ± 0.5 | nd | 8.6 ± 0.7 | nd |
| *Talaromyces ramulosus* (peach isolate) | 3.7 ± 0.4 | nd | 3.0 ± 0.1 | 3.6 ± 0.3 |
| *Talaromyces mineoluteus* (peach isolate) | 3.3 ± 0.2 | nd | 4.2 ± 0.2 | nd |
| *Penicillium expansum* (peach isolate) | 5.2 ± 0.3 | nd | 3.7 ± 0.3 | nd |
| *Penicillium digitatum* (citrus isolate) | 3.7 ± 0.2 | >20 | 3-4 | <20 |
| *Penicillium glabium* (wood isolate) | 9.8 ± 0.2 | nd | 4.0 ±0.5 | nd |
| *Trichoderma atroviride* (wood isolate) | 11 ± 0.4 | nd | 4.8 ± 0.3 | nd |
| *Phomopsis viticola* | nd | <10 | nd | <10 |
| *Phaeoacremonium aleophilum* | nd | <10 | nd | <10 |

| **Bacterial species** | | | | |
|---|---|---|---|---|
| *Bacillus subtilis* 168 | 12-20 | nd | 8-14 | nd |
| *Bacillus subtilis* OKB105 | 25-30 | nd | 8-14 | nd |
| *Bacillus subtilis* OKB120 | 12-20 | nd | 8-14 | nd |
| *Bacillus subtilis* ATCC21332 | 25-30 | nd | 8-14 | nd |
| *Bacillus spizizenii ATCC 6633* | 21 ± 3.9 | nd | 2.6 ± 0.2 | nd |
| *Micrococcus luteus* NCTC 8340 | 5.7 ± 0.11 | <10 | 28 ± 0.4 | 6 |
| *Listeria monocytogenes* B73 | 23 ± 0.63 | nd | 9.9 ± 0.2 | nd |
| *Listeria monocytogenes* B73-MR1 | 14 ± 0.30 | nd | 5.6 ± 0.1 | nd |
| *Escherichia coli* HB101 | > 100 | >100 | 44 | 40 |

In summary, the trials show that 3-12 mg/L (3-12 ppm or parts per million) of cyclic decapeptide eliminates up to 20 million fungal spores per liter of growth medium. Similarly, 15-25 mg/L (15-25 ppm) of cyclic decapeptide eliminates 2x10¹¹/L colony forming units of the bacterial food pathogen *L. monocytogenes.* From the results it is clear that the Trc mix and GS are capable of significant antimicrobial, and in particular antifungal, activity against a broad spectrum of pathogens.

The antifungal activities of the purified cyclic decapeptides, GS, TrcA₁, TrcA, TrcB₁, TrcB, TrcC₁, TrcC, PhcA, and TpcC were determined against *F. solani* and *B. cinerea* in both yeast extract modified tryptone soy broth (high salt medium, YTSB, results not shown) and potato dextrose broth (low salt PDB) (Table 2). Low µM activity was found in the high salt medium (YSTB) when compared to the low salt medium (PDB) for all the peptides, except for TpcC and PhcA. TpcC and PhcA were especially influenced by their environment, with TpcC exhibiting a decrease in activity of approximately twofold against *B. cinerea* and *F. solani* from YTSB to PDB and PhcA losing respectively ± 2 fold and ± 5 fold activities against *B. cinerea* and *F. solani* from PDB to YTSB.

**Table 2: Summary of the cyclic decapeptides and their antifungal activity against fungi grown in PDB. Standard one/three letter abbreviations are used with exception of O for ornithine, lower case letters for D-amino acids, (+) for cationic amino acid residue, Ar for aromatic amino acid residue.**

| **Identity** | **Abbr** | **Sequence** | **MIC against *B. cinerea* in PDB (n)** | **MIC against *F. solani* in PDB (n)** |
|---|---|---|---|---|
| Tyrocidine mixture | Trc Mix | Cyclo (Val-(+)-Leu-D-Phe-Pro-Ar-D-Ar-Asn-Gln-Ar) (SEQ ID NO: 178) | 4.8±0.7 (6) | 9.3±1.2 (3) |
| Tyrocidine C, | TrcC₁ | *Cyclo*-(VKLfPWwNQY) (SEQ ID NO: 179) | 8.9±1.5 (3) | 32±2.8 (3) |
| Tyrocidine C | TrcC | *Cyclo*-(VOLfPWwNQY) (SEQ ID NO:180) | 3.5±0.2 (6) | 4.3±0.4 (3) |
| Tyrocidine B₁/ B₁' | TrcB₁/B₁' | *Cyclo*-(VKLfP(W,f)NQY) (SEQ ID NO:) (SEQ ID NO: 181) | 3.6±0.1 (6) | 5.9±0.6 (3) |
| Tyrocidine B/B' | TrcB/B' | *Cyclo*-(VOLfP(W,f)NQY) (SEQ ID NO:182) | 5.1 ±1.0 (6) | 11±4.0 (3) |
| Tyrocidine A₁ | TrcA₁ | *Cyclo*-(VKLfPFfNQY) (SEQ ID NO: 183) | 4.1±0.7 (6) | 7.9±0.7 (3) |
| Tyrocidine A | TrcA | *Cyclo*-(VOLfPFfNQY) (SEQ ID NO: 184) | 5.2±0.6 (6) | 4.0±0.4 (3) |
| Tryptocidine C | TpcC | *Cyclo*-(VOLfPWwNQW) (SEQ ID NO: 185) | 3.6±0.2 (6) | 4.6±0.9 (3) |
| Phenycidine A | PhcA | *Cyc*/*o*-(VOLfPFfNQF) (SEQ ID NO: 186) | 6.9±1.4 (5) | 7.2±1.0 (3) |
| Gramicidin S | GS | *Cyclo*-(VOLfPVOIfP) (SEQ ID NO: 187) | 1.8±0.3 | 2.2±0.1 |

Overall, GS, TrcA and TrcC exhibited the highest activity with the most consistency against both *B. cinerea* and *F. solani* in YTSB and PDB. In general, the potent antifungal activities against both species in both mediums are good indications of salt tolerant antifungal activity that is necessary for protection of animal feed. Also, the cyclic decapeptide activity in liquid media is a prerequisite for control of fungal pathogens in, for example, hydration and hydroponic tanks that are used in plant propagation. Good activity was also found for both the Trc mix and GS on agar for a number of the target pathogens which is a good indication of their potential in surface sterilization. This was further explored and is discussed below.

### Antifungal activity on surfaces

Fungal species can be surface pathogens. For example, *B. cinerea* can be found on the surface of grapes (Ferreira,1990 S. Afr. J. Enol. Vitic 11, 38-41) and strawberries (Hang et al, 2005, Plant Pathol. J. 21, 59-63). Alternatively, pathogenic fungi such as *F. solani* can be found in soil (Booth, C. (Ed), 1971, The Genus Fusarium, Commonwealth Mycological institute, Surrey) or the xylem of plants (Alaniz et al., 2007, Plant Dis. 91, 1187-1193). Therefore, in order to protect fruit, seeds, cereals and dry food preparations, the antifungal agent must also be active in surface environments.

Only partial surface protection was afforded by the Trc mix against *F*. *solani,* which is not a major spoilage pathogen, while GS remained highly active against this pathogen grown on agar medium (Table 3). Both the Trc mix and GS were highly active against *B*. *cinerea* in both the PDA (potato dextrose agar) and YTSA (yeast extract modified tryptone soy agar) gel assays, similar to that in liquid media, showing surface activity (Table 3). This indicates that the Trc mix and GS could be used as protective agents for fruits against *B. cinerea,* a major fungal pathogen in fruit spoilage.

**Table 3: Summary of activity against F. solani and B. cinerea on PDA and YTSA of the Trc mix and GS.**

| | | PDA (µg/mL) | | YTSA (µg/mL) | |
|---|---|---|---|---|---|
| | | IC₅₀ ± SEM | MIC ± SEM | IC₅₀ ± SEM | MIC ± SEM |
| *F. solani* | Trc mix | 8.3 ± 3.7 | > 100 | 16.7 ± 0.8 | >100 |
| *B. cinerea* | | 5.0 ± 0.9 | 7.6 ± 1.7 | 7.44 ± 0.2 | 14.4 ± 1.5 |
| *F. solani* | GS | 4.9 ± 1.2 | 6.3 ± 1.5 | 9.6 ± 0.5 | 19.9 ± 1.2 |
| *B. cinerea* | | 2.0 ± 0.3 | 2.8 ± 0.3 | 4.5 ± 1.1 | 8.2 ± 1.8 |

Promising results in preliminarily trials on oranges have also been observed, with 50% and 80% less wound infections by *P. digitatum* using 50 mg/L (50 ppm) Trc mix and GS, respectively.

The Trc mix and GS showed strong antifungal activity against all three fungal pathogens tested in the presence and absence of wood cuttings (Table 4, Figure 1). The Trc mix was highly active against *Phomopsis* in the presence of grapevine stalk material. However, the Trc mix showed a reduced activity against *Cylindrocarpon* and even less so against *Phaeoacremonium* spores, when it was tested at 10 mg/L against these pathogens (Table 4). This would suggest that the Trc mix binds to the woody material, thus reducing the effective concentration required for killing certain fungal spores in solution, but potentially protecting the wood surface. This loss of activity in solution was, however, overcome by increasing the Trx mix concentration to 20 mg/L. It must also be emphasized that the Trc mix activity was tested at 10 times (10⁷ spores/L) the spore concentration used for the testing of commercial fungicides and at 100 times the spore concentration usually observed in contaminated grapevine material (personal communication with the Department of Plant pathology, Stellenbosch University).

**Table 4: The antifungal activity of the Trc mix and GS in the presence and absence of grapevine stalk (gvs) material under simulated hydration tank conditions. The percentage viable spores, as determined with a viability assay, compared to the growth/survival controls are indicated.**

| | ***Cylindrocarpon*** | | ***Phomopsis*** | | ***Phaeoacremonium*** | |
|---|---|---|---|---|---|---|
| **[Trc mix]** | **-gvs** | **+ gvs** | **- gvs** | **+ gvs** | **- gvs** | **+ gvs** |
| 10 µg/ml | 0 | 40 | 0 | 0 | 0 | 8 |
| 20 µg/ml | 0 | 0 | ND | ND | 0 | 0 |

| [Gram S] | - gvs | + gvs | - gvs | + gvs | - gvs | + gvs |
|---|---|---|---|---|---|---|
| 10 µg/ml | 0 | 0 | 0 | 0 | 0 | 0 |
| 20 µg/ml | 0 | 0 | ND | ND | 0 | 0 |

### In vivo antifungal activity

The Trc mix and GS were also tested for use in the sterilization of field-gathered grapevine material for use in plant cultures. It was found that 20 mg/L Trc mix or GS was sufficient to eliminate fungal pathogens in the washing step or in the growth media without a major detrimental effect to the plant material (results not shown). However, in selected cases, the antibacterial efficacy of the Trc mix was found to be inadequate, and in order to improve overall efficacy it had to be combined with GS, which showed good antibacterial activity. It was further found that 15 mg/L Trc mix or GS was sufficient to eliminate fungal pathogens in the growth media without a major detrimental effect to the plant material (Figure 1). The Trc mix not only enhanced plant culture survival, but significantly enhanced foliage, roots and overall growth above that of the control plants. GS also enhanced plant culture survival and foliage growth, but root growth was visibly affected. The Trc mix therefore shows great potential for sterilization, protection and overall growth stimulation of explants in growth media.

Field trials in which grapevine sticklings were treated after grafting with 30 mg/L Trc mix, GS or a mixture of GS and Trc mix showed that the treatment led to >15% higher survival of 2 month old sticklings in the vineyard (Figure 3). The treated sticklings also showed visibly more foliage, correlating to the grape plant culture results. This shows that the cyclic decapeptides alone or in combination also have efficacy in nursery conditions and also under agricultural conditions.

Cut flower trials on hybrids of the African daisy (*Gerbera hybrid*) showed that as little as 35 mg/L Trc mix, GS or GS+Trc mix in tap water substantially increases the water uptake (>45% improvement) above that of the flowers in water alone, indicating that opportunistic infections blocking the flower stem are suppressed. Water uptake was initially better than observed for a commercial product for cut-flower preservation (Figure 4A). The survival of the majority of the flowers was found to be ±2 days longer when treated with the Trc mix than flowers in untreated water control, GS or the GS+Trc mix, although slightly fewer flowers than the optimized commercial product were still in pristine condition (Figure 4B). GS at 35 mg/L alone or in the GS+Trc mix, however, did not benefit flower survival and may have led to some phytotoxicity in the Gerbera.

Trials with Delphinium flowers and 25 mg/L and 50 mg of GS or Trc mix and GS+Trc mix (50 mg/L combined concentration), were conducted. Trc mix at 25 and 50 mg/mL did not show phytotoxicity and preserved flowers better than controls in tap water. GS at 25 mg/L did not show overt phytotoxicity, but at 50 mg/L it again proved to be phytotoxic. The GS+Trc mix, however, which contained 25 mg/L GS compared to 35 mg/mL in the Gerbera trail, outperformed the commercial product in preservation of the Delphinium flowers (Figure 5). These results indicate that the Trc mix or an optimal combination of the GS + Trc mix have good potential in product formulations to extend the vase life of cut flowers as diverse as *Gerbera* spp. and *Delphinium* spp. in flower arrangements.

### Materials and methods

### Antifungal and antibacterial activity assays

The antibacterial activity of the tyrocidines and gramicidin S against all the bacteria tested was determined by the methods described by Du Toit EA and Rautenbach M (2000) J. Microbiol. Methods, 42, 159-165. The antifungal activity of the tyrocidines against all the bacteria tested was determined by the methods described by Troskie AM, Vlok NM and Rautenbach M (2012) J. Microbiol. Methods, 91, 551-558.

### Hydration tank assays

The activities of the GS and Trc mix were also assessed in the presence of and absence of grapevine stem material in a simulated hydration tank environment. Each 5.0 mL assay contained fungal spores at 10 000 spores/mL, peptide at 10 and 20 µg/mL and grapevine stem material. Grapevine stem material was washed with 70% ethanol and rinsed with sterile water before placement in inoculated hydration tanks, whereafter the Trc mix was added. Controls contained either the peptide or grapevine stem material. The assays were incubated at room temperature for 16 hours, whereafter three 100 µl aliquots of each assay were plated on PDA agar and allowed to grow for three days at 25°C. Some plates were stained with Coomassie brilliant blue stain (0.025% Coomassie, 12.5% isopropanol and 10% acetic acid) for better visualization of the fungal colonies. The number of colonies that developed after three days was recorded and expressed as percentage viable spores after comparing to the control samples which contained no peptide.

### Plant culture assays

Grapevine tissue culture plants were transferred to MS medium containing GS or Trc mix at a concentration of 15 µg/mL and incubated at 25°C with a 16h/8h day night cycle. The growth and development of the grapevine plantlets were monitored and compared to the control set of plants which were maintained on MS-medium without GS or Trc mix.

### Grape stickling field trials

Grape sticklings were treated after grafting by submersion of 2 x 500 sticklings/pail in 50 L water containing 30 mg/L Trc mix or 30 mg/L GS. Sequential submersion in 30 mg/L Trc mix and 30 mg/L GS was also conducted on 500 sticklings. Standardised nursery practice was followed and sticklings were incubated for three months before planting them out in a nursery vine yard. Dead sticklings and viable sticklings with foliage were counted after about one month of growth and compared with the control sticklings that were subjected to normal practice.

### Cut flower trials

Cut- flower- trials on mini hybrids of the African daisy (*Gerbera hybrid*) and blue larkspur (*Delphinium hybrid*) were conducted on 3-day old flowers. Flowers were prepared by cutting 3-5 cm of each stem and each placed separately in a measuring flask with 100 mL containing either no additive, Trc mix (35 mg/L for Gerbera and 25 mg/l for Delphinium), GS (35 mg/L for Gerbera and 25 mg/L for Delphinium), Trc mix + GS (combined 70 mg/L for Gerbera and combined 50 mg/L for Delphinium) or commercial additive for flower preservation. Flowers (6-12 per trial) were kept in a room with both natural and fluorescent lighting (16 hours light/day) at 22±1°C. The volume of water was monitored every 24 hours and noted for each flower.

Flower condition was rated from 0 to 5, with 0 = dead, 1 = flower petals dehydrated, closed and drooping, 2 = flower still partially open but dehydrated and drooping, 3 = flower starts drooping, petals soft, 4 = some petals becoming soft, flower still in good condition and 5 = flower is in pristine condition with taught petals and stem/leaves.

## Claims

1. A method of preventing or controlling microbial growth on plants, plant material, plant parts or in media used to sustain plants or plant material, the method comprising the step of applying an antimicrobial composition to the plants, plant material, plant parts or media,
wherein the antimicrobial composition comprises a cyclic decapeptide produced from *Bacillus aneurinolyticus* as an active agent, the cyclic decapeptide being a tyrocidine, tryptocidine or phenycidine or an analogue thereof comprising an amino acid sequence of *cyclo*(valine-X₁-leucine-D-phenylalanine-proline-X₂-X₃-X₄-X₅-X₆) (SEQ ID NO: 1), where:
X₁ is ornithine or lysine;
X₂ is valine, leucine, isoleucine, phenylalanine, tryptophan or tyrosine;
X₃ is the D-isomer of valine, leucine, isoleucine, phenylalanine, tryptophan or tyrosine;
X₄ is asparagine or glutamine;
X₅ is glutamine or asparagine; and
X₆ is tyrosine, phenylalanine or tryptophan.

2. A method of improving plant growth or plant vigour or prolonging the life of cut flowers, the method comprising the step of applying an antimicrobial composition to the plants, to plant material, plant parts or growth media, or to vase water for the cut flowers.
wherein the antimicrobial composition comprises a cyclic decapeptide produced from *Bacillus aneurinolyticus* as an active agent, the cyclic decapeptide being a tyrocidine, tryptocidine or phenycidine or an analogue thereof comprising an amino acid sequence of *cyclo*(valine-X₁-leucine-D-phenylalanine-proline-X₂-X₃₋X₄-X₅-X₅) (SEQ ID NO: 1), where:
X₁ is ornithine or lysine;
X₂ is valine, leucine, isoleucine, phenylalanine, tryptophan or tyrosine;
X₃ is the D-isomer of valine, leucine, isoleucine, phenylalanine, tryptophan or tyrosine;
X₄ is asparagine or glutamine;
X₅ is glutamine or asparagine; and
X₆ is tyrosine, phenylalanine or tryptophan.

3. A method according to either of claims 1 or 2, wherein the cyclic decapeptide is selected from the group consisting of the amino acid sequences shown in:
(a) any one of SEQ ID NOS: 6-59;
(b) any one of SEQ ID NOS: 60-113; or
(c) any one of SEQ ID NOS: 114-167.

4. A method according to any one of claims 1 to 3, wherein the antimicrobial composition contains more than one type of cyclic decapeptide of SEQ ID NO: 1.

5. A method according to any one of claims 1 to 4, wherein the antimicrobial composition contains an organism which produces the cyclic decapeptide.

6. A method according to any one of claims 1 to 5, wherein the microbial growth being controlled is fungal growth.

7. A method according to claim 6, which prevents or controls the growth of one or more fungi selected from the group consisting of *Phaeoacremonium* spp., *Phomopsis vilicola, Fusarium* spp., *Cylindrocarpon* spp., *Botrytis cinerea, Talaromyces* spp., *Aspergillus* spp. *Penicillium* spp., *Monilinia* spp., *Trichoderma* spp., and *Phaeomoniella* spp..

8. A method according to any one of claims 1 to 5, wherein the microbial growth being controlled is bacterial growth.

## Patentansprüche

1. Verfahren zur Verhinderung oder Bekämpfung von mikrobiellem Wachstum auf Pflanzen, Pflanzenmaterial, Pflanzenteilen oder in Medien, die zum Kräftigen von Pflanzen oder Pflanzenmaterial verwendet werden, wobei das Verfahren den Schritt des Aufbringens einer antimikrobiellen Zusammensetzung auf die Pflanzen, das Pflanzenmaterial, die Pflanzenteile oder die Medien umfasst,
wobei die antimikrobielle Zusammensetzung ein cyclisches Decapeptid umfasst, das aus *Bacillus aneurinolyticus* als Wirkstoff hergestellt wird, wobei das cyclische Decapeptid Tyrocidin, Tryptocidin oder Phenycidin oder ein Analogon davon ist, das eine Aminosäuresequenz von Cyclo(valin-X₁-leucin-D-phenylalanin-prolin-X₂-X₃-X₄-X₅-X₆) (SEQ ID NO: 1) umfasst, wobei:
X₁ Ornithin oder Lysin ist;
X₂ Valin, Leucin, Isoleucin, Phenylalanin, Tryptophan oder Tyrosin ist;
X₃ das D-Isomer von Valin, Leucin, Isoleucin, Phenylalanin, Tryptophan oder Tyrosin ist;
X₄ Asparagin oder Glutamin ist;
X₅ Glutamin oder Asparagin ist; und
X₆ Tyrosin, Phenylalanin oder Tryptophan ist.

2. Verfahren zum Verbessern von Pflanzenwachstum oder Pflanzenwachstumskraft oder Verlängern der Lebensdauer von Schnittblumen, wobei das Verfahren den Schritt des Aufbringens einer antimikrobiellen Zusammensetzung auf die Pflanzen, das Pflanzenmaterial, die Pflanzenteile oder die Wachstumsmedien oder das Vasenwasser für die Schnittblumen umfasst,
wobei die antimikrobielle Zusammensetzung ein cyclisches Decapeptid umfasst, das aus Bacillus aneurinolyticus als Wirkstoff hergestellt wird, wobei das cyclische Decapeptid Tyrocidin, Tryptocidin oder Phenycidin oder ein Analogon davon ist, das eine Aminosäuresequenz von Cyclo(valin-X₁-leucin-D-phenylalanin-prolin-X₂-X₃-X₄-X₅-X₆) (SEQ ID NO: 1) umfasst, wobei:
X₁ Ornithin oder Lysin ist;
X₂ Valin, Leucin, Isoleucin, Phenylalanin, Tryptophan oder Tyrosin ist;
X₃ das D-Isomer von Valin, Leucin, Isoleucin, Phenylalanin, Tryptophan oder Tyrosin ist;
X₄ Asparagin oder Glutamin ist;
X₅ Glutamin oder Asparagin ist; und
X₆ Tyrosin, Phenylalanin oder Tryptophan ist.

3. Verfahren nach einem der Ansprüche 1 oder 2, wobei das cyclische Decapeptid aus der Gruppe ausgewählt ist bestehend aus der Aminosäuresequenz, die gezeigt ist in:
(a) irgendeiner von SEQ ID NO: 6 - 59;
(b) irgendeiner von SEQ ID NO: 60 - 113; oder
(c) irgendeiner von SEQ ID NO: 114 - 167.

4. Verfahren nach einem der Ansprüche 1 bis 3, wobei die antimikrobielle Zusammensetzung mehr als einen Typ cyclisches Decapeptid von SEQ ID NO: 1 enthält.

5. Verfahren nach einem der Ansprüche 1 bis 4, wobei die antimikrobielle Zusammensetzung einen Organismus enthält, der das cyclische Decapeptid erzeugt.

6. Verfahren nach einem der Ansprüche 1 bis 5, wobei das antimikrobielle Wachstum bekämpftes Pilzwachstum ist.

7. Verfahren nach Anspruch 6, das das Wachstum von einem oder mehreren Pilzen verhindert oder bekämpft ausgewählt aus der Gruppe bestehend aus *Phaeoacremonium* spp., *Phomopsis viticola, Fusarium* spp., *Cylindrocarpon* spp., *Botrytis cinerea, Talaromyces* spp., *Aspergillus* spp. *Penicillium* spp., *Monilinia* spp., *Trichoderma* spp. und *Phaeomoniella* spp..

8. Verfahren nach einem der Ansprüche 1 bis 5, wobei das mikrobielle Wachstum, das bekämpft wird, Bakterienwachstum ist.

## Revendications

1. Procédé de prévention ou de contrôle de la croissance microbienne sur des plantes, une matière végétale, des parties de plantes ou dans des milieux utilisés pour entretenir des plantes ou une matière végétale, le procédé comprenant l'étape d'application d'une composition antimicrobienne aux plantes, à la matière végétale, aux parties de plantes ou aux milieux,
dans lequel la composition antimicrobienne comprend un décapeptide cyclique produit à partir de la souche *Basilus aneurinolyticus* comme agent actif, le décapeptide cyclique étant une tyrocidine, une tryptocidine ou une phénycidine ou un de leurs analogues comprenant une séquence d'acides aminés de cyclo(valine-X₁-leucine-D-phénylalanine-proline-X₂-X₃-X₄-X₅-X₆) (SEQ ID NO: 1), où :
X₁ est l'ornithine ou la lysine ;
X₂ est la valine, la leucine, l'isoleucine, la phénylalanine, le tryptophane ou la tyrosine ;
X₃ est le D-isomère de la valine, de la leucine, de l'isoleucine, de la phénylalanine, du tryptophane ou de la tyrosine ;
X₄ est l'asparagine ou la glutamine ;
X₅ est la glutamine ou l'asparagine ; et
X₆ est la tyrosine, la phénylalanine ou le tryptophane.

2. Procédé d'amélioration de la croissance de plantes ou de la vigueur de plantes ou du prolongement de la durée de vie de fleurs coupées, le procédé comprenant l'étape d'application d'une composition antimicrobienne aux plantes, à une matière végétale, aux parties de plantes ou aux milieux de croissance ou encore à l'eau du vase pour les fleurs coupées ;
dans lequel la composition antimicrobienne comprend un décapeptide cyclique produit à partir de la souche *Basilus aneurinolyticus* comme agent actif, le décapeptide cyclique étant une tyrocidine, une tryptocidine ou une phénycidine ou un de leurs analogues comprenant une séquence d'acides aminés de *cyclo*(valine-X₁-leucine-D-phénylalanine-proline-X₂-X₃-X₄-X₅-X₆) (SEQ ID NO: 1), où :
X₁ est l'ornithine ou la lysine ;
X₂ est la valine, la leucine, l'isoleucine, la phénylalanine, le tryptophane ou la tyrosine ;
X₃ est le D-isomère de la valine, de la leucine, de l'isoleucine, de la phénylalanine, du tryptophane ou de la tyrosine ;
X₄ est l'asparagine ou la glutamine ;
X₅ est la glutamine ou l'asparagine ; et
X₆ est la tyrosine, la phénylalanine ou le tryptophane.

3. Procédé selon l'une ou l'autre des revendications 1 ou 2, dans lequel le décapeptide cyclique est choisi dans le groupe constitué des séquences d'acides aminés présentées dans :
(a) l'une quelconque des SEQ ID NO: 6 à 59 ;
(b) l'une quelconque des SEQ ID NO: 60 à 113 ; ou
(c) l'une quelconque des SEQ ID NO: 114 à 167.

4. Procédé selon l'une quelconque des revendications 1 à 3, dans lequel la composition antimicrobienne contient plus d'un type de décapeptide cyclique de SEQ ID NO: 1.

5. Procédé selon l'une quelconque des revendications 1 à 4, dans lequel la composition antimicrobienne contient un organisme qui produit le décapeptide cyclique.

6. Procédé selon l'une quelconque des revendications 1 à 5, dans lequel la croissance microbienne contrôlée est la croissance fongique.

7. Procédé selon la revendication 6 qui empêche ou contrôle la croissance d'un ou plus de champignons choisis dans le groupe constitué des suivants :
*Phaeoacremonium* spp., *Phomopsis viticola, Fusarium* spp., *Cylindrocarpon* spp., *Botrytis cinerea, Talaromyces* spp., *Aspergillus* spp., *Penicillium* spp., *Monilinia* spp., *Trichoderma* spp. et *Phaeomoniella* spp.

8. Procédé selon l'une quelconque des revendications 1 à 5, dans lequel la croissance microbienne contrôlée est la croissance bactérienne.
